# EUROPEAN PATENT SPECIFICATION

(11) **EP 1 059 882 B1**
(45) Date of publication and mention of the grant of the patent: **17.10.2007**
(21) Application number: 99911184.2
(22) Date of filing: 05.03.1999
(51) Int. Cl.: A61B 10/00, A61B 5/00, A61K 41/00, A61M 37/00, G01N 33/50

(54) **INTEGRATED TISSUE PORATION, FLUID HARVESTING AND ANALYSIS DEVICE**
INTEGRIERTE GEWEBEPORATIONS-, FLÜSSIGKEITSAMMEL- UND ANALYSEVORRICHTUNG
DISPOSITIF INTEGRE DE FORMATION DE PORES, DE PRELEVEMENT DE LIQUIDE BIOLOGIQUE ET D'ANALYSE

(30) Priority: 06.03.1998 US 77135 P
(43) Date of publication of application: 20.12.2000
(73) Proprietor: SpectRx, Inc., Norcross, GA 30071 (US); Altea Therapeutics Corporation, Tucker, Georgia 30084 (US)
(72) Inventor: EPPSTEIN, Jonathan, A., Atlanta, GA 30345 (US); SAMUELS, Mark, Norcross, GA 30092 (US); HATCH, Michael, R., Sugar Hill, GA 30518 (US)
(74) Representative: Thomson, Paul Anthony
(86) International application number: PCT/US1999/004983
(87) International publication number: WO 1999/044507

(56) References cited:
- WO-A-97/07734
- WO-A-98/00193
- US-A- 5 458 140

## Description

### BACKGROUND OF THE INVENTION

### Field of the Invention

This invention relates to an integrated device for the creation of micropores or small holes or perforations in tissue, the collecting a biological fluid from the tissue through the small holes, and monitoring or analyzing a characteristic of the biological fluid, such as for the concentration of an analyte.

### Discussion of the Art

The prevalence of diabetes has been increasing markedly in the world. At this time, diagnosed diabetics represent approximately 3% of the population of the United States. It is believed that the total actual number of diabetics in the United States is much higher. Diabetes can lead to numerous complications, such as, for example, retinopathy, nephropathy, and neuropathy.

The most important factor for reducing diabetes-associated complications is the maintenance of an appropriate level of glucose in the bloodstream. Proper maintenance of the level of glucose in the bloodstream may prevent and even reverse many of the effects of diabetes.

Traditional glucose monitoring devices operate on the principle of taking blood from an individual by a variety of methods, such as by needle or lancet. This is a multiple step process. First, a needle or lancet is used to make a hole in the individual's skin deep enough to obtain blood. Next, the individual applies a drop of blood to a strip which contains chemistry that interacts with the blood. Finally, the strip is inserted into a blood-glucose meter for measurement of glucose concentration based on a change in reflectance of the strip.

These traditional glucose monitoring systems require that an individual have separately available a needle or lancet for extracting blood, separately available strips carrying blood chemistry for creating a chemical reaction with respect to the glucose in the blood stream and changing color, and a blood-glucose meter for reading the change in color indicating the level of glucose in the bloodstream. The level of blood glucose, when measured by a glucose meter, is read from a strip carrying the blood chemistry through a well-known process.

There are other technologies being developed to provide an alternative to the conventional blood glucose monitoring procedures. One such technology involves measuring the level of glucose in interstitial fluid. In order to obtain samples of interstitial fluid, the barrier function of the stratum corneum must be overcome.

Published PCT application WO 9707734 entitled "Microporation Of Human Skin For Drug Delivery and Monitoring Applications," to Eppstein et al., discloses a method of ablating the stratum corneum to form at least one micropore by treating a selected area of the stratum corneum with an effective amount of an optically absorbing compound, such as a dye, that exhibits strong absorption over the emission range of a pulsed light source and thermally ablating the stratum corneum by optically heating the dye. Heat is conductively transferred by the dye to the stratum corneum to elevate the temperature of tissue-bound water and other vaporizable substances in the selected area above the vaporization point of water and other vaporizable substances. Another microporation technique disclosed in that application involves the use of a solid thermal probe that is applied directly to the tissue. To the individual, these techniques are much less painful than using a lancet, if not completely painless.

Another technique for removing the stratum corneum is by direct absorption of optical energy. See, for example, U.S. Patent No. 4,775,361 to Jacques et al.

In sum, there are several ways of making small holes in the tissue including breaching the tissue mechanically with a needle or lancet, removing layers of tissue by thermal ablation techniques described above, or by the direct absorption of optical energy.

There is room for improving glucose monitoring technologies. In particular, it is desirable to integrate several functions of the glucose monitoring procedure into a single device. Preferably, this device would facilitate the harvesting of a biological fluid, such as interstitial fluid, by making one or more small holes in the tissue, and the analysis of the biological fluid to determine a measure of a characteristic of the biological fluid, such as glucose level.

US Patent 5,458,140 discloses a method of enhancing the permeability of the skin or mucosa to an analyte so that such can be used for diagnosis purposes.

### SUMMARY OF THE INVENTION

Briefly, the present invention is directed to an integrated device for harvesting biological fluid, such as interstitial fluid or blood, from tissue and analyzing the biological fluid. According to the present invention there is provided an integrated fluid harvesting and analysis device as claimed in claim 1, Several embodiments of an integrated device are disclosed. It is submitted that all disclosures provided hereinafter, which do not fall within the scope of the device claimed in claim , are provided for information purposes only and are not intended to extend the scope of protection afforded to the present invention.

### BRIEF DESCRIPTION OF THE DRAWINGS

FIG. 1 is a comprehensive diagram of an analyte assay system including an integrated device according to one embodiment of the present invention.
FIG. 2 is a comprehensive diagram of an analyte assay system including an integrated device according to another embodiment of the present invention.
FIG. 3 is a partial cross-sectional view of the integrated device according to the first embodiment showing how biological fluid is collected and delivered to an assay pad.
FIG. 4 is a top view showing the arrangement of the sense electrodes and sensor of the integrated device according to the first embodiment.
FIG. 5 is a side view of the integrated device showing the application of a deformation force to the integrated device of the first embodiment.
FIG. 6 is a comprehensive diagram of an analyte assay system and showing a cross-sectional view of an integrated device according to still another embodiment of the present invention.
FIG. 7 is a side cross-sectional view of the integrated device shown in FIG. 6.
FIG. 8 is a perspective view of a hand-held assay unit and integrated device associated therewith according to still another embodiment of the present invention.
FIG. 9 is a perspective view of the integrated device according shown in FIG. 8.
FIG. 10 is a cross-sectional view taken through line 10-10 of FIG. 9.
FIG. 11 is a cross-sectional view of an integrated device according to another embodiment of the present invention.
FIG. 12 is a bottom view of the integrated device of FIG. 11.
FIG. 13 is a side cross-sectional view of an integrated device having a sensor which is optically read.
FIG. 14 is a top view of an integrated device that is optically read.
FIG. 15 is a top view of a pneumatic sealing system used in conjunction with an integrated device.
FIG. 16 is a side view of a pneumatic sealing system shown in FIG. 15.
FIG. 17 is an enlarged side view of the use of a mechanical pressure device with an integrated device.
FIG. 18 is an enlarged side view showing the effects of the mechanical pressure device shown in FIG. 17.
FIGs. 19 and 20 are schematic diagrams showing the application of sonic energy in conjunction with the integrated device.
FIG. 21 is a side view of an integrated device according to another embodiment of the invention in which the poration elements are mechanical porating elements.
FIG. 22 is a partial perspective view showing the integrated device of FIG. 21 in greater detail.

### DETAILED DESCRIPTION OF THE DRAWINGS

### Definitions

As used herein, the expression "biological fluid" is intended to include blood serum or whole blood as well as interstitial fluid. "Interstitial fluid" is the clear fluid that occupies the space between the cells in the body. The term "stratum corneum" means the outermost layer of the skin, consisting of from about 15 to about 20 layers of cells in various stages of drying out. The stratum corneum provides a barrier to the loss of water from inside the body to the external environment and from attack from the external environment to the interior of the body. The term "epidermis" means the metabolically active region of the skin. It is found just below the stratum corneum and is approximately 10 times as thick as the stratum corneum. The epidermis does not contain blood transport structures, i.e., capillaries. The term "dermis" means the region of skin approximately 10 times as thick as the epidermis and found just below the epidermis. The dermis contains large amounts of collagen, which provides structural integrity to the skin. The dermis contains a layer of small blood capillaries that provide oxygen and nutrients to the rest of the layers of skin.

As used herein, the term "tissue" means an aggregate of cells of a particular kind, together with their intercellular substance, that forms a structural material. At least one surface of the tissue must be accessible to electromagnetic radiation so that one embodiment of the invention can be carried out. The preferred tissue is the skin. Other tissues suitable for use with this invention include mucosal tissue and soft organs.

As used herein, "ablation" refers to the process of controlled removal of a selected area of tissue from the surrounding tissue by kinetic energy released when the temperature of vaporizable substances in the selected area is rapidly elevated above the vaporization point thereby flash vaporizing some of the tissue in the selected area.

As used herein, "poration," "microporation," or any such similar term means the formation of a small hole, opening or pore to a desired depth in or through a biological membrane, such as skin or mucous membrane, or the outer layer of an organism to lessen the barrier properties of this biological membrane to the passage of biological fluids, such as analytes from within the biological membrane or the passage of permeants or drugs from without the biological membrane into the body for selected purposes, or for certain medical or surgical procedures. The size of the hole or "micropore" so formed is approximately 1-1000µm in diameter. It is to be understood that the term "micropore" is used in the singular form for simplicity, but that it multiple openings or pores may be formed by the integrated device according to the present invention.

As used herein, the term "integrated device" means a device suitable for forming small holes or micropores in tissue, collecting a biological fluid from the tissue (preferably through the micropores so created) and analyzing the biological fluid to determine a characteristic thereof.

As used herein, "sonic energy" refers to mechanical pressure waves with frequencies from 10 Hz to 1000 MHz.

The term "porating element" is meant to include any means of forming a micropore, hole or opening described above, including by thermal ablation, mechanically breaching the tissue by lancet or needle, and other known techniques. An example of a mechanical porating element is disclosed in commonly assigned published PCT application WO 98/00193, entitled, "Multiple Mechanical Microporation Of Skin Or Mucosa".

The term "heated probe" or "heat conducting element" means a probe, preferably solid phase, which is capable of being heated in response to the application of electrical or electromagnetic (optical) energy thereto for achieving thermal ablation of the tissue. For simplicity, the probe is referred to as a "heated probe" or "heatable probe" which includes a probe in a heated or unheated state, but which is heatable.

Several embodiments of the integrated device are disclosed herein. In each of the embodiments, a porating element is provided that is used to form at least one opening in the tissue and in the layer that is in contact with the skin. In some embodiments, the porating element is a heated probe or heat conducting element which, when heated, forms at least one opening, i.e. a micropore, in the tissue. What is common among these embodiments is that the heated probe is heated such that the temperature of tissue-bound water and other vaporizable substances in a selected area of the surface of the tissue, such as the stratum corneum, is elevated above the vaporization point of water and other vaporizable substances thereby removing the surface of the tissue in the selected area. Consequently, the heated probe forms a micropore in the surface of the tissue approximately 1-1000 µm in diameter.

Some of the microporation techniques described herein are further described in published PCT application WO 97/07734.

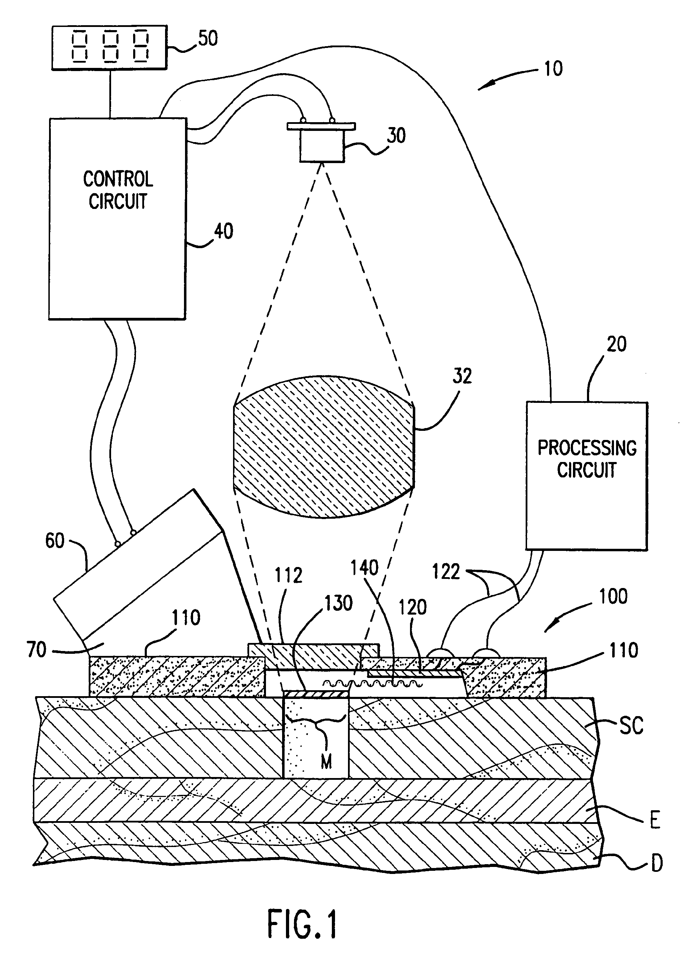

Referring first to FIG. 1, an analyte assay system is shown at reference numeral 10 comprising an integrated device 100. The configuration of the integrated device 100 is shown in a simplified manner so as to illustrate several basic elements of the inventive integrated device. The integrated device 100 comprises a substrate layer 110 that includes an optically transparent window 112 on at least a portion thereof. An analyte sensor 120 is disposed on an under-surface of the substrate layer 110. In the embodiment of the invention where the analyte sensor 120 is an electrochemical biosensor, the integrated device 100 has electrode leads 122 that connect to the analyte sensor 120 and to a processing circuit 20.

A layer of photothermal material 130 is provided on the bottom surface of the substrate 110 or directly applied to the tissue surface from which biological fluid is to be collected. In addition, a layer of adhesive may be applied to certain bottom surfaces of the substrate 110 to hold the integrated device onto the tissue surface and prevent biological fluid from being drawn between the tissue surface and the bottom layer of the integrated device 110. The integrated device 100 could be a one-use disposable element, or could be suitable for multiple uses. The photothermal material layer 130 also serves to seal the bottom surface of the integrated device 100 to protect the analyte sensor 120 from the external environment. Suitable compounds for the photothermal material are described in the aforementioned published PCT application, WO 97/07734, and in U.S. Provisional Application No. 60/077,135. Alternatively, a hole or opening may be provided at the location of the optically transparent window 112, and a quantity of photothermal material is disposed directly on the tissue surface, or on a bottom surface of the integrated device 100.

The integrated device 100 and all other specific embodiments described hereinafter, are designed to form micropores in tissue, collect fluid from the tissue, and analyze the fluid in a single (integrated) step. As an example, FIG. 1 shows a stratum corneum layer SC and epidermis E of skin. The micropores may be of a depth that extends into the stratum corneum SC, or may extend through the stratum corneum to (and into) the epidermis layer E. The micropores may go still further through the epidermis layer E into the vascularized dermal layer to obtain whole blood as the fluid sample, rather than just interstitial fluid.

In operation, optical energy from a source 30, such as a laser diode, is projected through the optical window 112 of the integrated device 100. A focusing lens 32 may be provided to focus the beam onto the integrated device 100. The optical energy from the source 30 is focused onto the layer of photothermal material 130. The photothermal material 130 heats up in response to absorbing the optical energy and transfers heat to the surface of the tissue. Once sufficient heat is transferred to the surface of the tissue, a micropore is formed in the tissue, such as into the stratum corneum SC. In the process, the photothermal material 130 vaporizes together with the layers of stratum corneum that are affected by the heat conducted from the optically heated photothermal material 130. Consequently, the micropore M formed in the tissue creates a path into the integrated device 100. Similar size openings are formed in the photothermal material 130 at the bottom portion of the integrated device 100 so as to permit flow of fluid into the integrated device 100.

More specifically, the micropore M can permit interstitial fluid in the tissue to flow into the integrated device 100 and eventually to contact the sensor 120. The sensor 120 then reacts to the interstitial fluid to measure a concentration of an analyte, such as glucose. The processing circuit 20 is any well known glucose measuring circuit that is capable of measuring the output of an electrochemical analyte sensor and producing a reading correlated to the concentration of a target analyte in biological fluid, such as glucose.

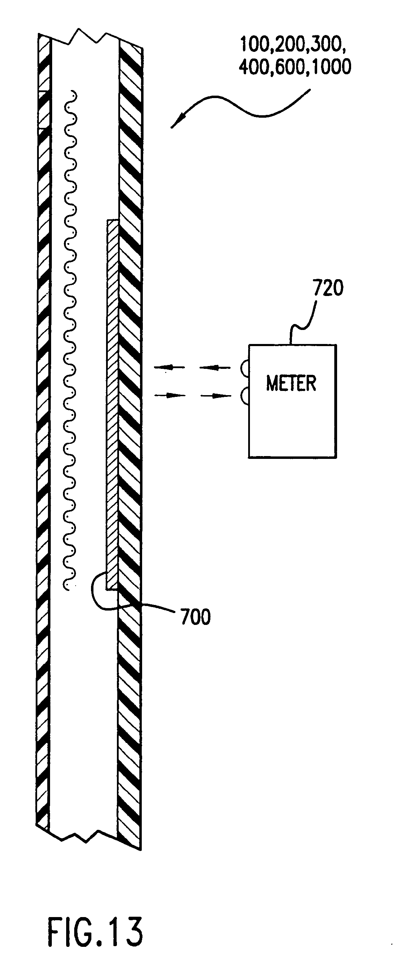

Alternatively, as shown in FIG. 13, a colorimetric assay system may be employed, instead of the electrochemical one shown in the version of the integrated device of FIG. 1. The colorimetric assay system used in conjunction with an integrated device is described hereinafter in conjunction with FIG. 13.

A control circuit 40 may be provided that is connected to the source 30 and to the processing circuit 20. The control circuit 40 may further process the assay measurement made by the processing circuit 20 to drive a display 50 in order to display the assay measurement. In addition, as an optional enhancement, sonic energy may be applied to the microporated tissue by a sonic transducer 60 that is coupled to the tissue by a portion of the substrate layer 110 that is made of suitable acoustically coupling material. The sonic transducer 60 may be a piezoelectric device, a magneto-restrictive device or a small electromagnetic transducer, such as a miniature audio speaker element of a moving coil, moving magnet or electrostatic design. The sonic energy coupled to the tissue acts as a driving force to direct interstitial fluid into the integrated device 100. Moreover, the sonic transducer 60 may be comprised of one or more separate elements each of which may be individually controlled to achieve different effects on the focus and energy density of the sonic energy within the system.

The sonic energy may be focused or formed by a beam former 70. The beam forming may be achieved via a combination of one or more elements 70 which are placed in the propagation path of the sonic waves emitted by the transducer 60. By selectively altering the propagation velocity of the sonic waves, these elements can be shown to produce a redirection of this energy and if desired a focusing can be achieved, similar to the way diffractive elements in an optical lens system effect the light waves passing through them. A plano-concave aluminum element may be placed on the surface of the transducer. The radius of the concave cutout nominally defines the focal point of the system. Alternatively, a focus of sonic energy may be achieved by using multiple sonic sources operated in a coordinated fashion to form a phased array wherein energy peaks and nulls are defined by the additive superpositioning of the sonic energy waves.

With a sonic energy system, one can also easily create a standing wave pattern wherein the natural resonance of the system creates localized stationary energy peaks. Once a standing wave has been established, only a small amount of additional energy is needed to maintain it at this same amplitude. Also, subtle perturbations in the system such as a small shift in frequency or a separate action which affects the systems natural resonance can cause the standing wave to move in a controllable and predictable fashion allowing manipulation of the fluid sample as desired. Using multiple transducers, one can also establish a standing wave and easily control the position, amplitude and period between wave peaks.

Further, vacuum (negative pressure) may be applied to the microporated site to assist in the harvesting of the biological fluid into the integrated device so as to make contact with the sensor. Likewise, positive pressure may be applied to the integrated device 100 with a downward force on the integrated device 100 to force fluid to move towards the sensor 120. The application of positive pressure is described in more detail hereinafter.

To enhance the flow of the fluid to the sensor 120, the surface tension effects may employed. For example, surfactant compounds are optionally applied to surfaces of the integrated device 100 to direct fluid flow to the sensor. Furthermore, a mesh 140 may be provided in the integrated device 100 to wick interstitial fluid towards the sensor 120. The mesh 140 is positioned and clamped between top and bottom layers of the integrated device, or may be held in place by small thermal welds, glue, or mechanical spacers. The mesh 140 acts by a surface tension mechanism to move the biological fluid to the sensor. Still further, a capillary channel may be formed between the top and bottom layers of the integrated device 100, thereby creating surface tension effects to move the fluid to the sensor 120.

The mesh 140 may be treated with a surfactant compound as well. Further still, surfaces of the integrated device 100 where it is desired that interstitial not flow may be treated with hydrophobic compounds. The mesh 140 will also displace volume in the integrated device to thereby reduce the volume of interstitial fluid needed for an adequate assay measurement. The technique of treating a wicking mesh layer with surfactants to transport a fluid to an assay sensor is known in the art. See, for example, U.S. Patent No. 5,271,895 to McCroskey et al. Other examples of known uses of surfactant treated layers are disclosed in U.S. Patent Nos. 3,992,158 to Przybylowicz et al., 4,050,898 to Goffe, deceased et al., 3,912,457 to Ogawa et al., 4,053,381 to Hamblen et al., 4,774,192 to Terminiello et al., and 4,839,296 to Kennedy et al.

Still further, the sensor 120 may be a type that has modified surface tension properties achieved by treatment with a surfactant compound. Such sensors are well known in the art, and include assay pads or strips manufactured and distributed by Medisense, Boehringer Mannheimn, Kyoto Dai-ichi (KDK), Miles-Bayer, and Lifescan. Specifically, by way of example only, and not by limitation, electrochemical and colorimetric strips made by Medisense, Boehringer Mannheimn, Miles have proven suitable. Likewise, electrochemical strips made by KDK and the colorimetric strip made by Lifescan are also suitable. A specific example of a sensor that is treated with a surfactant is the Elite strip manufactured and sold by Miles-Bayer.

In practice, the sensor used in the integrated device according to the present is smaller in size than the assay strips traditionally used in blood-glucose monitoring systems.

Examples of thickness dimensions for the various components of the integrated device are as follows.

| Element | Thickness (Microns) |
|---|---|
| Optical Window | 20-1000 |
| Sensor & Electrodes | 5-200 |
| Mesh | 20-400 |
| Photothermal Layer | 20-100 |
| Sonic Coupling Portion of Substrate | 100-1000 |
| Complete Assembly | 160-2600 |

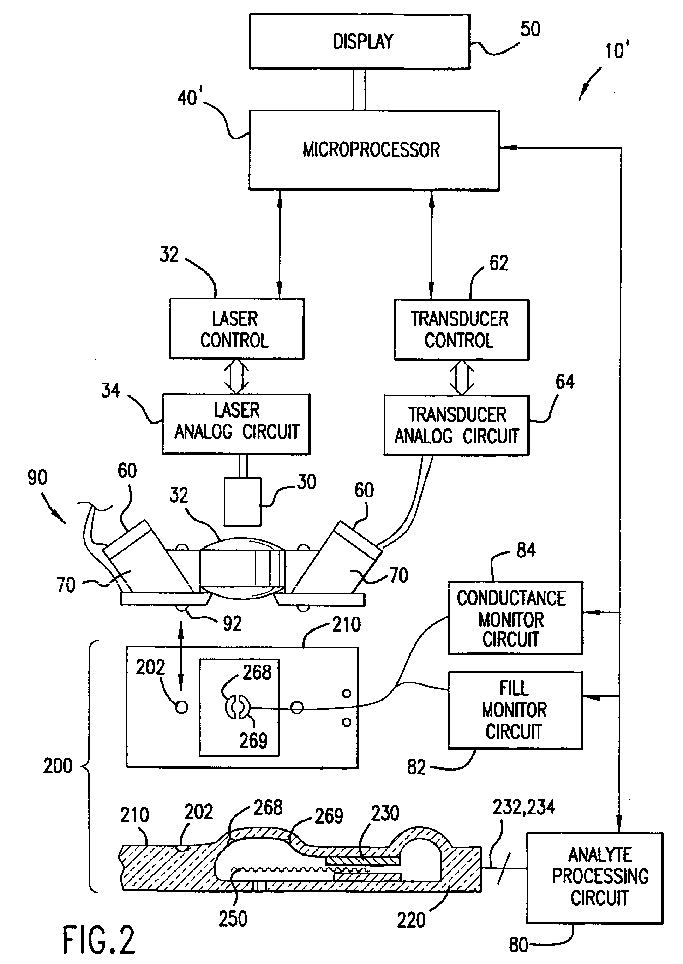

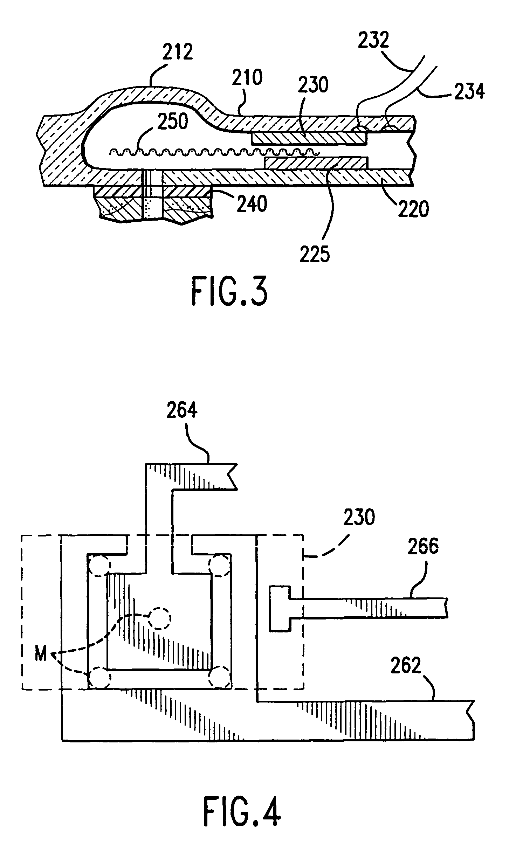

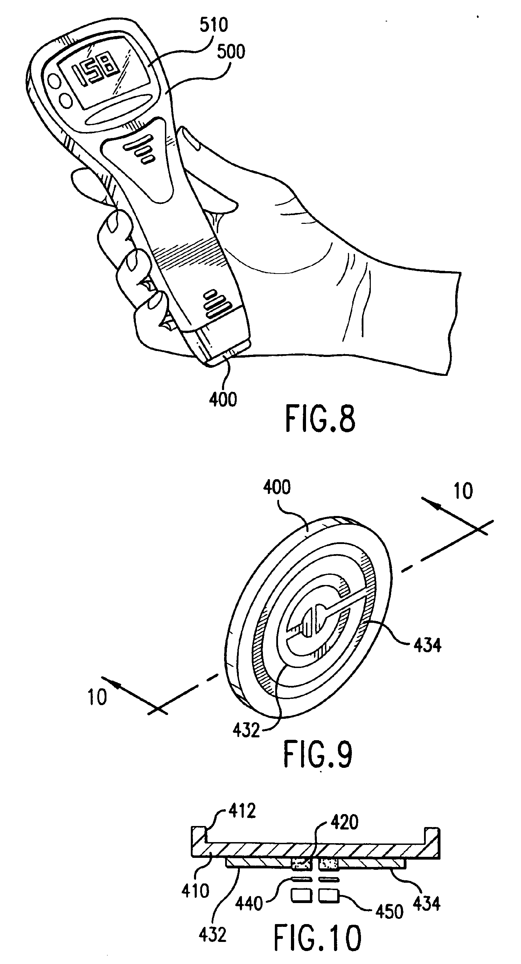

Turning to FIGs. 2-4, an integrated device 200 according to another embodiment is shown. The integrated device 200 is shown as part of an analyte assay system that is similar to system 10' shown in FIG. 1, but with additional features. The integrated device 200, the details of which are best shown in FIGs. 3 and 4, comprises a top layer 210, a bottom layer 220, and a sensor 230. The top layer 210 may be integral with the bottom layer 220. The top layer 210 has an optically transparent window portion 212. The sensor 230 is disposed between the top layer 210 and the bottom layer 220. A layer of photothermal material 240 is either applied to the tissue, or is formed on or integrally with the bottom layer 220. Electrode leads 232 connect to the sensor 230. Electrical connections to the integrated device 200 are preferably made with a position-invariant type of contact allowing for easy installation and removal of the integrated device 200 from a electrochemical sensor meter and/or optical energy source. FIG. 9 illustrates an embodiment that achieves this in a concentric configuration.

An optional mesh layer 250 is provided in the integrated device to direct collected biological fluid to the sensor. The mesh layer 250 may be treated with a surfactant compound as explained above in conjunction with FIG. 1.

To assist in defining the exact volume of fluid presented to the assay chamber and the sensor 230 a spacer element 225 is placed between the bottom layer 220 and the top layer 210. The spacer element 225 specifies the volume of fluid within the active area of the sensor 230. Specifically, if the aspect ratio of the height (H) of the spacer 225, to the width (W) of the roughly square or circular sensor 230 is less than about 0.1, then for all practical purposes the useable volume of fluid presented to the sensor 230 is defined by H*W*W.

For some embodiments of the present invention, it may be useful to form the micropores some lateral distance away from the sensor 230. In such a case, a surface tension driven or capillary feed channel provides an efficient and volumetrically compact method for conducting the flow of the sampled fluid from the micropore to the sensor 230. This channel may be optionally filled with a mesh layer 250 similar to that traditionally used in a number of existing glucose monitoring strips manufactured by both Boehringer-Mannheim and Medisense as well as others in the industry. As described above, both the capillary channel and the optional mesh layer 250 may be treated with a surfactant compound to enhance the fluid conductivity along this channel.

As shown in FIG. 4, there are two electrodes 262 and 264 that extend in and around the sensor 230, as is well known in the art. The electrode lead 232 connects to electrode 262 and electrode lead 234 connects to electrode 264. Electrodes 262 and 264 are the sense electrodes. Electrodes 268 and 269 shown best in FIG. 2, are the fill electrodes and are connected to the conductance monitor circuit 84 and to the fill monitor circuit 82.

Alternatively, one of the fill electrodes 268 and 269 could be shared with one of the sense electrodes 262 and 264. For example, if a third electrode 266 were strategically placed to the right of electrode 262, and the fill direction was defined to be from the left, then when conductance was sensed between electrodes 262 and 266, this would indicate that the sensor was fully wetted and the reading process can be initiated via the sense electrodes 262 and 262. Similarly, if both a conductance monitor circuit 84 and a fill monitor circuit 82 are incorporated as well as an electrochemical sense system, typically a common anode or common cathode design could be used as one leg of each of these circuits to reduce the total number of electrical traces necessary to be run into the integrated device.

In some forms of the integrated device 200, the conductance monitor circuit 84 is essentially the same as the fill monitor circuit 82, but the conductance monitor is responsive to the very first signs of a fluid sample in the integrated device. This can be used to control the poration process and shut it off in a closed loop fashion as soon as an active pore has been formed as determined by the ability of the pore to source fluid. However, the fill monitor circuit 82 is responsive to determining that the sensor 230 has been sufficiently wetted with a fluid sample in order to begin the measurement of the sensor 230 by the analyte processing circuit 80. The electrode leads 232 and 234 connect the sense electrodes 262 and 264 to the analyte processing circuit.

A microprocessor control circuit 40' is provided, and is connected to the analyte processing circuit 80 and fill monitor circuit 82. The microprocessor control circuit 40' is programmed to control the interaction with the integrated device 200. The microprocessor control circuit 40' generates signals to display analyte measurements and other information on the display 50. In addition, the microprocessor control circuit 40' controls the application of optical energy and other parameters to the integrated device 200 to effect the microporation and harvesting process. For example, if after the harvesting cycle has begun and some preset amount of time has elapsed during which the fill circuit has not detected a sufficient amount of fluid delivered to the sensor, a suitable error message might be displayed and the user prompted to reinitialize the system, install a new integrated device 200 and try again at a fresh site on the tissue surface. As a further enhancement, a temperature measurement may be obtained from the site and/or sensor in order to correct for temperature sensitivities in the measurements obtained by the sensor.

To this end, the system 10' includes a laser control circuit 32, a laser analog circuit 34, a transducer control circuit 62 and a transducer analog circuit 64. Alternatively, both the digital control circuitry and analog output stages could be combined into a single circuit or even fabricated as a mixed mode application specific integrated circuit (ASIC). With an ASIC implementation it would also be possible to incorporate all of the master controller microprocessor circuitry, all of the display drivers circuitry, and any other input-output circuitry all on the single ASIC chip, yielding a much simpler, potentially lower cost and more reliable system. The following discussions of the particular functions of each portion of the control and driver circuitry apply equally to either the discrete implementation, a partially integrated ASIC version or a wholly integrated ASIC version.

The laser control circuit 32 is responsive to commands from the microprocessor control circuit 40' to generate analog signals that are processed by the laser analog circuit 34 to drive the optical source 30. Similarly, the transducer control circuit 62 is responsive to commands from the microprocessor control circuit 40' to generate analog signals that are processed by the transducer analog circuit 64 to drive the sonic transducer 60.

An interface assembly 90 supports the sonic transducer 60, beam former 70 and focusing lens 32. The interface assembly further comprises an alignment member 92 that mates with an alignment indentation or key 202 on the substrate layer 210 of the integrated device 200. This assures that the optical energy from the source 30 will be properly focused on the integrated device 200, and that sonic energy will be properly coupled through the integrated device 200 to the tissue. These alignment features also ensure that a proper reference is achieved between the micropore formed and the fluid sample collected, and further facilitates proper wetting of the sensor 230 inside the integrated device.

The operation of the system 10' and integrated device 200 is as follows. Once the integrated device 200 is in position on the surface of the tissue, the microprocessor control circuit 40' activates the source 30. The source 30 may be a pulsed laser diode, and the microprocessor control circuit 40' will activate it within a predetermined energy range. The optical energy is focused onto the tissue surface through the substrate top layer 210 and onto the photothermal material 240. The photothermal material 240 is responsive to the optical energy to transfer heat to the surface of the tissue to form one or more micropores therein. As shown in FIG. 4, the one or more micropores M are shown as being made around the periphery or in the middle of the sensor 230. In tests conducted, it was observed that a series of pores placed on one side of the assay pad, under a clear cover layer, effectively formed a capillary feed channel into the area of the assay pad and achieved a uniform wetting of the assay pad as the fluid front swept across it, wetting it without bubbles. Placing the fill sensor electrodes on the side opposite from this fill direction would generally assure that when the fill indicator was tripped, the assay pad could be used to correctly assay the fluid.

The microprocessor 40' may also control the application of sonic energy. The application of optical energy and/or sonic energy continues until the conductance monitor circuit 84 senses the presence of some amount of biological fluid in the integrated device 200. When the conductance monitor circuit 84 detects the presence of biological fluid in the integrated device, the optical source 30 is deactivated. However, the microprocessor 200 may continue the delivery of sonic energy until the fill monitor circuit 82 detects that the integrated device 200 has collected sufficient biological fluid to make an accurate assay measurement, or until some maximum time period expires. Once this occurs, the biological fluid will contact the sensor 230 and an analyte measurement can be made by the analyte measurement circuit 80, which information is coupled to the microprocessor control circuit 40' for display on the display 50. The optional wicking mesh layer 250 (optionally treated with a surfactant compound) will assist in directing the biological fluid to the sensor.

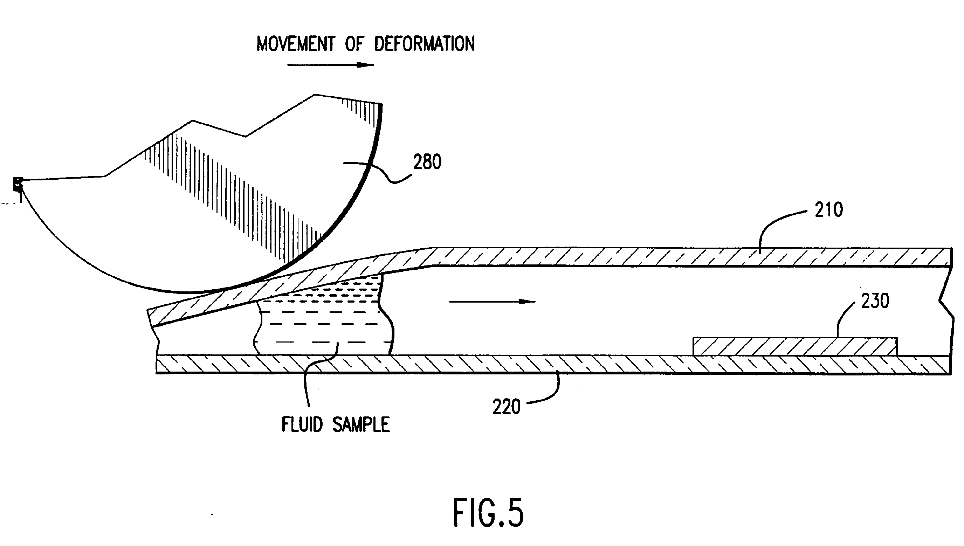

Another way to direct the biological fluid to the sensor 230 is by applying a mechanical force downward on the integrated device 200, as shown in FIG. 5. A cam or roller mechanism 280 is applied to the top of the integrated device to form an indent in the integrated device 200 along the top of the layer forming the upper boundary of a capillary channel between the top layer 210 and bottom layer 220. By letting the channel fill to some degree optionally until a fill sensor indicated a sufficient amount has been collected, the cam mechanism 280 is applied in a "squeegee" type action, moving the fluid sample down this channel to the sensor 230. This allows a positive, rapid delivery of fluid to the sensor with a minimum amount of fluid sample.

Furthermore, the application of negative pressure may be used to harvest the biological fluid into the integrated device 200. It has been shown in clinical studies that a small level of pressure reduction, even as little as ¼ ATM, can produce a steady outflow of interstitial fluid from the micropores. The flux rate under vacuum appears to obey an essentially linear relationship with pressure, as the pressure is reduced to 1 ATM, however, optimal values seem to be more in the ¼ to ¾ ATM range due to effects on the surrounding tissue and potential heating of the fluid sample.

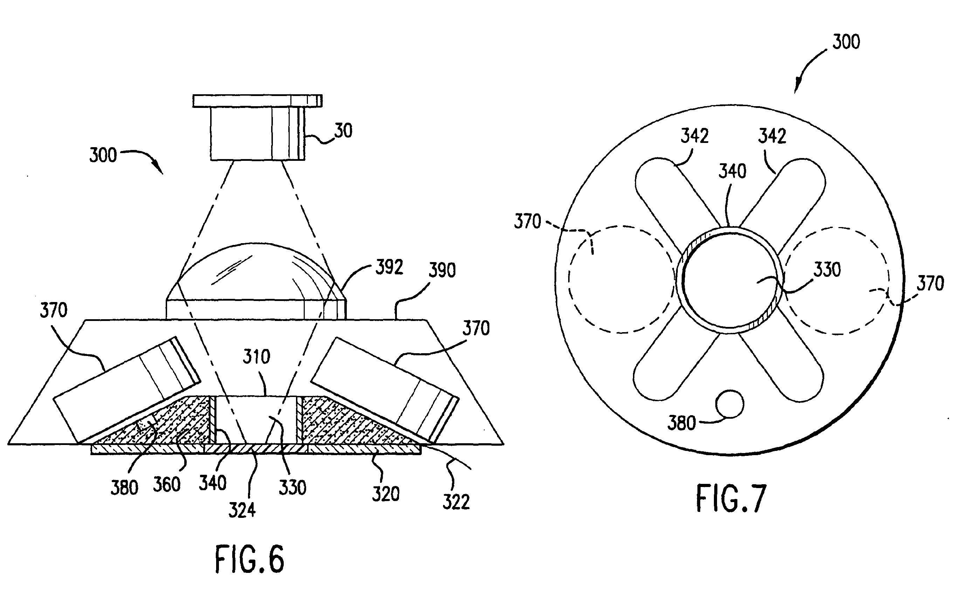

Turning to FIGs. 6-7, an integrated device 300 according to another embodiment is described. The integrated device 300 has a different configuration than integrated devices 100 and 200 described above. Integrated device 300 has a trapezoidal cross-section and comprises an optically transparent top membrane 310 and a bottom layer 320. A removable membrane 322 may be provided that covers the bottom layer 320 until the integrated device 300 is to be positioned on tissue for use. At least a portion of the bottom layer 320 may be treated with a photothermal material 324. Optionally, the membrane 322 is not removable and comprises a thin film of an optically absorbent photothermal material designed to vaporize during the microporation process. According to still another alternative, the tissue itself may be treated with photothermal material before the integrated device 300 is positioned on the tissue. The bottom layer 320 would be made of optically transparent material that vaporizes in the presence of the thermal energy created during poration, or this portion of the bottom layer may be removable just prior to applying the integrated device to the pre-treated area of the tissue surface.

Further, at least a portion of the bottom layer may be treated with an adhesive so that the integrated device 300 is secured to the surface of the tissue. The adhesive serves several functions. First, it preserves proper registration of the openings created in the integrated device with the openings created in the tissue to collect the fluid sample. Second, it allows for attachment of the device to the tissue so that an individual can operate it hands-free. Third, the adhesive will form a vacuum seal between the bottom layer of the integrated device and the tissue surface, thereby preventing biological fluid from passing beneath the integrated device uncollected. The vacuum seal also facilitates the application of negative pressure to the harvesting site.

The integrated device 300 comprises a chamber 330 to collect biological fluid in the center portion of the device. The chamber 330 is cylindrical in shape as shown in FIGs. 6 and 7. A sensor 340 is disposed on at least a portion of the inner wall of the chamber 340. Although FIGs. 6 and 7 show a cylindrical chamber, any other shape convenient to a particular application may be used for the chamber. For example, if the integrated device is manufactured in large quantities, a flat walled shape, such as a triangle, square or pentagon, in which the active sensor pad is placed on one of these walls.

As best shown in FIG. 7, a typical electrical wiring diagram is shown to support most electrochemical sensors used to date. There are an anode 342 and cathode 344 that connect to the sensor 340 each of which extends outward from the center of the integrated device 300. In addition, a reference electrode 352 and a sense electrode 354 are provided that connect to the sensor 340.

Optionally, the integrated device 300 may include additional electrodes to support the "fill" and "conductance" features described above, or even if the electrochemical assay system used requires additional electrical interfaces to function optimally. The reference electrode is useful for many electrochemical assays to provide a self calibrating feature wherein the actual assay reaction can be read as more of a difference measurement across a balanced impedance bridge. The sense electrode could be the assay output, which is similar to the "fill" or a "conductance" signal described above. Although dedicated anode and cathode terminals are shown, it is common practice to use one or the other of these as the sense electrode and share the other one with the reference electrode. The electrode configuration shown in FIGs. 6 and 7 indicates that the integrated device 300 supports the use of many of electrode configurations.

Disposed around the chamber 330 between the top membrane 310 and the bottom layer 320 is an acoustic lens 360 formed of material suitable for coupling sonic energy. For example, the acoustic lens 360 is formed of silicone material molded into a shape suitable for being disposed inside the integrated device 330. A lens material of a suitable durometer value will ensure sufficient sonic coupling to the tissue beneath, and also achieves pneumatic sealing if suction or negative pressure is used in the harvesting process. Sonic transducers 370 are positioned on the lateral surfaces of the integrated device to deliver sonic energy to the tissue through the acoustic lens 360. In order to facilitate alignment of the integrated device 300 with the remaining components of an assay system (similar to that shown in FIGs. 1 and 2), there is a reference hole 380 provided on the surface of the integrated device 300. The integrated device 300 may be contained within a housing 390 that includes an optical focusing lens 392 molded therein to focus optical energy from source 30 onto the photothermal material 324.

The operation of the integrated device 300 is similar to the descriptions above of integrated devices 100 and 200. However, the biological fluid harvested by the integrated device 300 is collected in the chamber 330 and contacts the sensor 340 placed on the side walls of the chamber 330, rather than a planar sensor shown in the previous embodiments.

Turning to FIGs. 8-10, an integrated device 400 according to a third specific embodiment will be described. The integrated device 400 is designed for use with a hand-held unit 500 that processes assay measurements obtained by the integrated device 400 and displays the measurements on a display 510.

The integrated device 400 is a disc-shaped member that supports the components for fluid harvesting and assay measurement. The integrated device 400 comprises a substrate member 410 configured in a circular shape and having flanges 412 that snap fit onto a bottom portion of the hand-held unit 500. A sensor 420 is positioned at a central location on one surface of the integrated device 400. First and second electrodes (anode and cathode) 432 and 434 extend concentrically on the bottom surface of the substrate 410 and make connection from opposite sides to the sensor 420. An optional mesh 440 may be disposed over the sensor 420. The mesh 440 may be treated with a suitable surfactant compound described above. A layer of photothermal material 450 is disposed over the opening placed on the bottom of the integrated device to allow the harvested fluid to wet the sensor 420, or the tissue itself is treated with photothermal material 450.

The integrated device 400 is operated by attachment to the hand-held unit 500 and positioned on the surface of the tissue to be microporated. The hand-held unit contains the optical source to focus optical energy onto the photothermal layer 450 to form one or more micropores in the tissue. Biological fluid from the tissue makes contact with the sensor 420 (preferably with the assistance of the mesh layer 440). The hand-held unit 500 includes processing circuitry that electrically couples to the electrodes 432 and 434 to obtain an assay measurement from the sensor 420. The hand-held unit 500 is activated by engaging the integrated device 400 against the tissue surface with sufficient pressure together with pressing an activation button on the hand-held unit 500.

In the previous embodiments of the integrated device, the poration process is based on the application of optical energy to an absorber target which in turn heats up sufficiently to conductively deliver enough thermal energy to the skin to ultimately cause the desired thermally induced microporation. An alternative approach to delivering this heat energy to the poration sites involves the placement of an electrically heated probe directly at the poration site. The temperature of the electrically heated probe is modulated as needed to effect the microporation process.

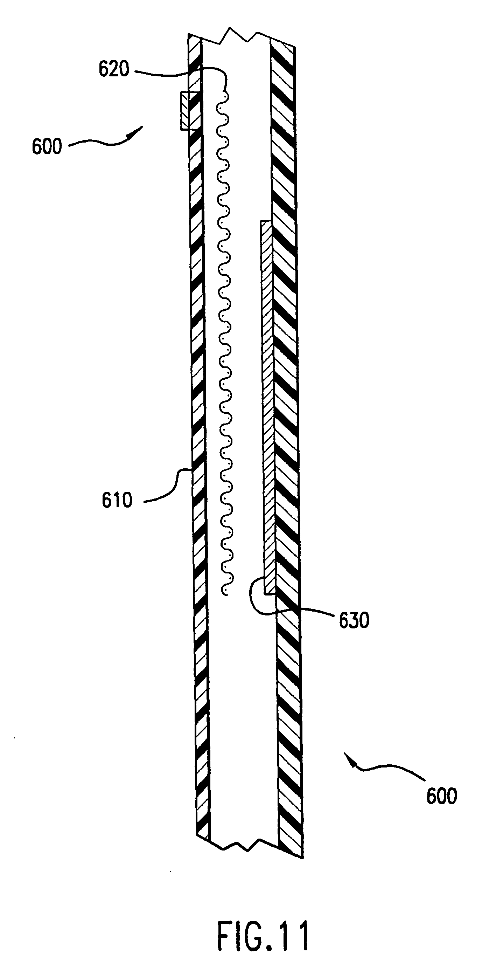

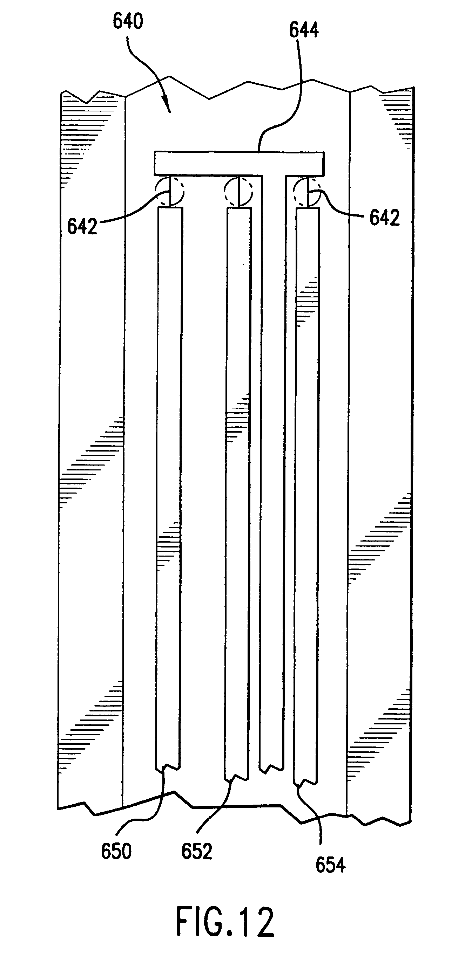

A schematic representation of an integrated device 600 employing an electrically heated probe (heat conducting element) is shown in FIGs. 11 and 12. The integrated device 600 comprises a layer 610, an optional mesh layer 620, and a sensor 630, which in this example, is a colorimetric sensor. It should be understood, however, that this same concept could easily be modified to employ the electrochemical biosensor. Moreover, as described in the foregoing, many of the aspects of the assay/fluid management systems of the device are optional, such as the use of the mesh layer 620, surfactant treated portions of the fluid management chamber, optically transparent windows in the layers to allow the reading of a colorimetric assay, methods for applying sonic energy, vacuum or negative pressure, mechanical manipulation, etc.

In the integrated device 600, there is provided at least one electrically heated probe 640. The types of electrically heated probes that are suitable are disclosed in the aforementioned published PCT application, WO 97/07734.

As shown in more detail in FIG. 12, the electrically heated probe 640 comprises an electrically conductive element or wire 642 provided on the bottom surface of the layer 12. Three electrically conductive elements 640 are shown as an example, though any number of them may be provided. An electrical conductor 644 extends the length of the layer 610 and terminates in a "T" that extends laterally across one end of the layer 610. Three other electrical conductors, 650, 652 and 654 extend the length of the layer 610 and terminate at a plurality of points near the termination of conductor 644. The three elements 640 are connected to conductor 642 and respectively to conductors 650, 652 and 654.

The electrical conductors 644, 650, 652 and 654 required to activate the elements 640 (also called poration elements hereinafter) can be made through the same type of connectors used to interface to the electrical output electrochemical biosensor. Each poration element 640 can be activated individually through the appropriate selection and energization of the conductors 650, 652 and 654. It may be advantageous to excite all poration elements 640 simultaneously, thereby enabling either a series or parallel wiring design, reducing the number of interconnections to the disposable poration system and facilitating a more rapid poration process. If only one element 640 is provided, then at least two conductors are provided for supplying electric current through the heatable element. One of these conductors may be shared with the assay sensor/fill/conductance circuitry as a common anode or cathode, thereby necessitating the additional of only one electrical connection to the integrated device.

These electrically activated thermal poration elements could be installed on a conventionally manufactured assay strip as an additional post-processing step. Preferably, the conductors 644, 650, 652 and 654 are embedded within the tissue-contacting layer so as not to be exposed on the bottom surface thereof, but to enable sufficient electrical connection to the one or more heated elements 640.

Each of the elements 640 functions as a solid thermal probe and is electrically heated so that the temperature of the tissue, if skin, is raised to a temperature greater than 123 C. For example, each element comprises a 100 to 500 micron long 50 micron diameter tungsten wire. These tungsten wires are typically laid flat against some form of backing (such as the tissue-contacting layer 12) which naturally limits the depth of penetration of the wire into the tissue (by virtue of the diameter of the wire). The temperature of the wire may be modulated according to the techniques disclosed in the aforementioned PCT publication.

The inlet ports to the fluid management chamber of the integrated device 600 may be small holes in the tissue-contacting layer across which the wires 640 extend. Alternatively, a meltable or vaporizable membrane is placed above the wires 640. When energized, the wires melt a hole in this membrane, creating an inlet port to the fluid management chamber at each location of the wires 640.

A system can be designed wherein the electrically heated poration elements 640 are contained in a separate component or device, which may be reusable. These elements would be replaced when it is detected that they are worn sufficiently to require replacement, or routinely, such as on a weekly basis, similar to a diabetic subject's replacement of a lancet tip in a fingertip lancing blood-drawing device.

In all of the foregoing embodiments of the integrated device an assay system according to the present invention, the type of optical energy source may be any of those described in the aforementioned PCT application WO 97/07734. Likewise, the types of substances used for the photothermal material are disclosed in PCT publication WO 97/07734 and the aforementioned U.S. provisional application.

In the foregoing embodiments of the integrated device, the sensor used to react with the harvested biological fluid and measure a characteristic of the fluid may be an electrochemical biosensor comprised of a layer or layers of chemicals capable of reacting with an analyte in a collected biological fluid to produce a measurable electrical response (as specifically shown in some of the foregoing embodiments). U.S. Patent Nos. 4,545,382 and 4,711,245 describe detecting layers capable of generating a measurable electrical signal in response to glucose in blood. The electrical signals are measured by a measuring circuit (such as the one shown in FIGs. 1 and 2 at reference numerals 20 and 80, respectively) obtained by electrical leads connected to electrodes in or around the active area of the biosensor. Alternatively, the sensor may be a colorimeter sensor, a fluorescent intensity-based sensor or a fluorescent life-time based sensor. Examples of electrochemical biosensors that are suitable for use in the integrated device are those manufactured by Medisense, Boehringer Manheim, KDK, etc.

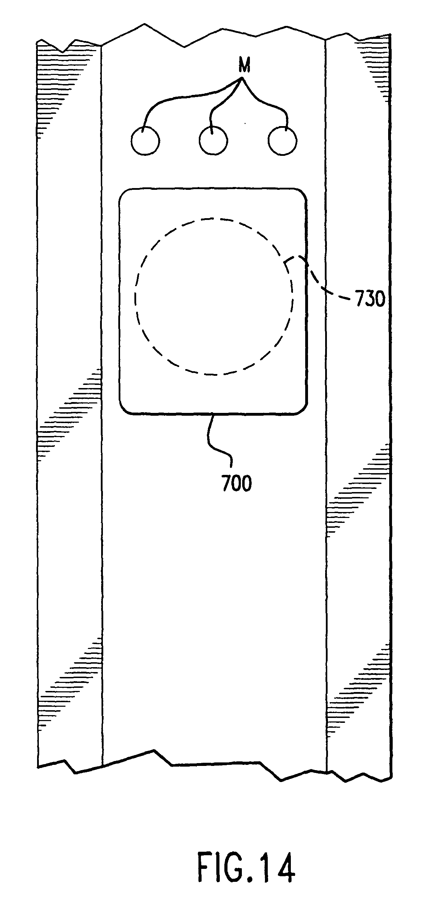

If a colorimeter sensor, a fluorescent intensity-based sensor or a fluorescent life-time based sensor is used, the sensor is "read" by an arrangement shown in FIGs. 13 and 14. The integrated device shown in FIG. 13 may be any one of those described in the foregoing embodiments. The sensor to be optically read is shown at reference numeral 700. The sensor 700 is held in sufficient registration to enable the optical field of view 730 of an optical meter 720 to be placed nominally in the center of the region of the colorimetric sensor wetted by biological fluid. The field of view of the optical meter 720 is optically cropped such that it conservatively under fills the area known to be wetted by the fluid sample. This reduces both the precision required during manufacture of the integrated device and the degree of initial and maintained registration of the integrated device on the meter and the individual, thus reducing cost and increasing reliability. In addition, this reduces the actual volume of biological fluid required to produce an accurate reading of the amount of the selected analyte present in the biological fluid. Colorimetric sensor technology for measuring glucose concentration is well known in the art. Furthermore, examples of fluorescent based sensor technology are disclosed in U.S. Patent Nos. 5,660,991; 5,631,169; 5,624,847; 5,504,337; 5,485,530 and 5,281,825, all to Lakowicz et al.

Specifically, it is a standard in the field of disposable assay strips to completely wet an area of the reagent treated portion of the assay strip much larger, typically 5 to 10 times larger, than the total area actually read by the meter. This practice allows relaxation of manufacturing tolerances in many parts of the system. This is also a common feature in the "fingerstick" blood-based glucose monitoring systems due to the physical difficulty of the user placing a smaller sample only on the actual target spot as well as the need for most whole blood-based systems to separate the corpuscular components from the serum. By incorporating the automatic registration of the micropores M with the sensor 700 through the design of the device, the assay process can be conducted accurately with a much smaller sample of the fluid than the typical fluid based disposable assay technology currently available.

If the assay technique used in connection with the integrated device is based on a fluorescent intensity technology, the colorimetric sensor 700 is treated with a probe fluorophore. A reaction between a probe fluorophore and the selected analyte produces a predictable change in the fluorescent intensity of the probe molecules when excited with a particular optical wavelength such that the subsequent fluorescence is detected at a selected longer wavelength. Optionally, the fluorescent probe is selected such that it can emit in two different wavelength bands, wherein the intensity of energy in only one of the bands is predictably modified by the varying concentration of the selected analyte. A ratiometric processing of the two different fluorescent intensities can be employed, thereby simplifying the calibration of the reading and allows for self-adjustment for different amounts or areas of the colorimetric sensor wetted with the biological fluid. Moreover, the fluorescent interrogation field of view may be defined by the intersection of the incident excitation light and the look field of the fluorescent receive channels.

Further still, the assay technique used in conjunction with the integrated device may be based on a fluorescent lifetime based assay technology. In this case, a reaction between a probe fluorophore, with which the colorimetric sensor 700 is treated, and the selected analyte produces a predictable change in the fluorescent lifetime of the probe molecules when excited with a particular wavelength. The subsequent fluorescent lifetime is detected at a selected longer wavelength. The detection of the fluorescent lifetime may be accomplished by either measuring directly the decay of the fluorescence in response to a known pulse shape of excitation light, or by measuring the phase shift and modulation depth of the fluorescent signal in response to the excitation of the sensor by a periodic modulated light source at the appropriate excitation wavelength. By basing the quantification of the analyte on a time resolved measurement, much of the difficulty associated with the calibration of an absolute intensity based measurement is overcome. Also, the signal-to-noise aspects of such a system are easily optimized. For example, in a phase detection system, it is routine to integrate for a sufficient period of time in order to resolve the phase to any level needed. Consequently, very small amounts of the probe molecule and biological fluid are required to achieve the desired level of quantification of the selected analyte, yielding additional benefits in the potential reduction of the required biological fluid sample volumes to the levels of only a few hundred nanoliters.

The integrated device according to the present invention may be used in a continuous monitoring system. The system could be integrated with other devices, including an insulin pump. The continuous monitoring system would provide a real-time feedback to achieve a closed loop artificial pancreas system without requiring an implant. The integrated device would be connected to a "smart" insulin pump that would respond initiate a glucose measurement (or one would be initiated on demand by the patient) and would administer an appropriate amount of insulin depending on the glucose measurement obtained.

In all of the foregoing embodiments on the integrated device, each has a fluid management chamber designed to direct the biological fluid collected to the sensor. The surfaces of the fluid management chamber may be selectively treated with chemical substances, such as a wicking agent, or a surfactant to induce the migration of fluid in a particular direction, i.e., to the sensor. Alternatively, certain portions of the surfaces of the layers in the fluid management chamber, such as the tissue-contacting layer, may be treated with a hydrophobic compounds or substances to direct the biological fluid away from a selected region or regions where it is not desired for the biological fluid to migrate and to direct the biological fluid toward the sensor. In the continuous monitoring embodiments described above, additional fluid management considerations may have to be given to deal with the "waste" fluid which would be created as a fresh fluid sample where harvested and moved into the sensor area. One solution for this waste fluid is to merely expand the overflow region provided in many of the current assay strip designs discussed and referenced above, to a size large enough to act as a fluid sink for this used fluid sample. This expanded overflow region also addresses the desire to keep these fluid samples contained within the integrated device element to maintain control over the ultimate disposal of the biological sample such that it can be handled with appropriate caution.

Furthermore, by designing the integrated device in such a manner that the biological fluid management is handled with minimal dead space outside of the active region of the sensor, a system can be built which uses very small samples of biological fluid to obtain an accurate assay of a selected analyte. Tests have been conducted on commercially available systems using glucose sensing amperometric biosensors that incorporated all of these features and it was found that the glucose concentration in a sample of biological fluid smaller than 1/3 of a microliter could be quantified, by modifying commercially available glucose test strips. One of the additional advantages gained by using interstitial fluid as the fluid sample for the assay system is the almost total lack of red blood cells in the sample. Most commercial blood-strip based assay systems utilize some means of separating the corpuscular component from a whole blood sample prior to the application of the fluid sample to the assay element. In many cases, this process is performed by the use of some sort of wicking mesh designed to trap the blood cells and let only the serum move through to the assay area. These filtering approaches can use up as much as 4/5 of the original sample volume in the process. By using interstitial fluid, this step is no longer needed. In other words, a typical sample size of 3 to 10 microliters is normally required for a blood based glucose monitoring disposable assay strip design whereas by utilizing the ability to place an unfiltered interstitial fluid sample directly on the active reagent treated portion of an assay system, it has been demonstrated that quantitative readings of a selected analyte can be obtained with fluid samples as small as 1/3 µL of interstitial fluid using modified conventional disposable assay strip technologies.

Another example of a closed lop application of the integrated device is to collect and monitor the levels of a particular drug in the blood stream or other fluid, wherein it is desired to maintain the drug level within a defined serum concentration window. In this case, an infusion pump would be controlled to respond, taking into account the total system bandwidth, and deliver small pulses of the therapeutic drug into the subject until the desired set point level had been reached, wherein the pump would then be put on standby until the drug levels dropped below the set point, thus triggering another pulse of the drug. Bandwidth in this context refers to the sum of all delays associates with the infusion of the drug, distribution within the body, diffusion into the fluid reservoirs from which the integrated device collects its fluid sample, and any additional delays in the sampling and processing involved before a change in the reported assay value would occur. Several drugs commonly prescribed could benefit from this sort of tight control over dosage levels such as many of the anti-seizure drugs, pain medications, chemotherapies, etc. In the case of pain medication, an additional control input could be given to the user allowing them to ask for an additional dose in an on-demand fashion to deal with breakthrough pain, and use the closed loop monitoring feature as a final safety to ensure that no toxic overdose levels would occur.

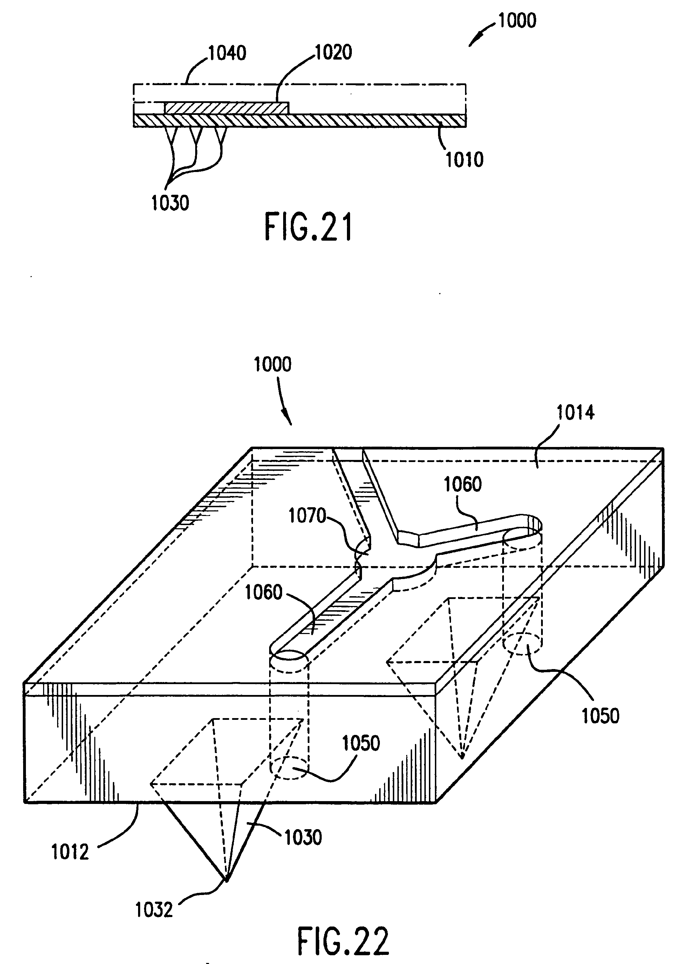

Turning to FIGs. 21 and 22, an integrated device 1000 according to still another embodiment is shown. The integrated device 1000 is similar in many respects to the previous embodiments, but includes one or more mechanical porating elements. Specifically, the integrated device 1000 comprises a substrate layer 1010 and a plurality of mechanical porating elements 1030 protruding from the bottom of the substrate layer 1010. The specific structure and arrangement of the mechanical porating elements are disclosed in commonly assigned published PCT application WO 98/00193, referred to above. The integrated device 1000 further comprises an assay sensor 1020 disposed above the mechanical porating elements 1030. The assay sensor 1020 is any of the sensors described above. An optional additional top layer 1040 may be provided to seal the top surface of the integrated device. If the assay sensor 1020 is a type that is optically read, then the top layer 1040 is optically transparent.

The mechanical porating elements 1030 are puncturing elements very small in size (10 to 50 microns), and are spaced apart from each other. With reference to FIG. 22, each porating element 1030 comprises a sharp point or edge 1032 for puncturing the tissue surface. Depending on the desired depth of the micropores to be created, the height of the porating element 1030 will vary. The porating elements 1030 may be pyramid or wedge shaped, which is easily created by microfabrication techniques, such as microlithography. Other shapes for the porating elements 1030 may be suitable, such as that of micro-lancets or micro-needles.

There are pluralities of holes 1050 extending from the lower side 1012 of the substrate layer 1010 on which the porating elements 1030 are exposed, to the upper side of the substrate layer 1014. Each porating element 1030 is adjacent to and paired with at least one hole for collecting biological fluid that seeps out of the punctured tissue. The holes 1050 are of suitable dimension to permit biological fluid, such as blood or interstitial fluid, to move by capillary action from the lower side 1012 of the substrate layer 1010 to the upper side 1014. The holes 1050 may be interconnected with channels 1060 that are formed on the upper side 1014, and the channels 1060 may intersect at a reservoir 1070. The assay sensor 1020 (not shown in FIG. 22 for simplicity) is then positioned on top of, partially overlapping, or adjacent the reservoir 170, so that it is sufficiently wetted with the biological fluid to make a suitable measurement.

As in the foregoing embodiments, the integrated device 1000 may include surface tension enhancements, such as a wicking mesh (surfactant treated or not) and surfactant treatment of the holes 1050 and channels 1060. The wicking mesh would be positioned to overlie the reservoir 1070 and thereby enhance the transport of the biological fluid to the assay sensor 1020. Furthermore, the integrated device 1000 may be modified to include any of the enhancements discussed below.

### Enhancements To Integrated Device

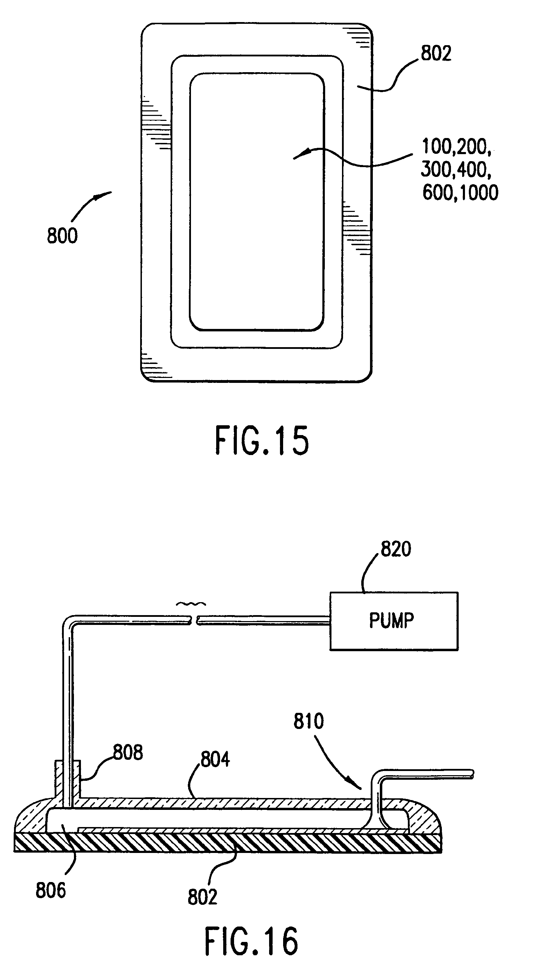

FIGs. 15 and 16 illustrate the use of a pneumatic seal together with any one of the integrated devices described above. A sealing means in the form of a sealing assembly 800 is provided which comprises a perimeter base 802 that fits around the integrated device (100, 200, 300, 400, 600, 1000), and a top layer 804 that is sealed to the perimeter base 802, and extends above the integrated device. The sealing assembly 800 pneumatically seals around the integrated device to the surface of the tissue. If the integrated device is of the type that requires exposure to optical energy, the top layer 804 is made of optically transparent material. The perimeter base 802 seals to the tissue surface around the integrated device, such as by an adhesive, or a tacky silicone, rubber or plastic element. A sealed chamber 806 is formed in the space between the integrated device and the top layer 804. A vacuum port 808 is provided in the top layer 804 for connection to a means for supplying negative pressure, such as a pump 820 or other source of negative pressure, such as a syringe, a diaphragm or some portion of the chamber which can be flexed outward to increase the volume of the chamber and thereby reduce the pressure within the chamber or the like. In addition, if an integrated device is used that requires connection to an electrode on the sensor and/or probe, this connection is made through a sealed electrical connector 810 in the top layer 804.

The sealed chamber 106 is formed against the surface of the tissue, such as the skin, over the poration site(s). The pressure in the chamber 106 can be reduced to provide a positive pressure gradient from within the body towards the sealed chamber 106 through the micropores to induce the biological fluid to exit the body and enter the integrated device 10 more rapidly.

By maintaining the total internal volume of the chamber 806 as small as possible, only providing the needed clearance for the integrated device, the evaporative losses of the biological fluid can be minimized. Once the humidity inside the chamber 806 reaches a saturation point, no more evaporative losses can occur. These evaporative losses can further be reduced by managing the biological fluid in a manner wherein the exposed surface area of the biological fluid pool that has exited the tissue is kept small. When induced to enter the device, the biological fluid is constrained on all sides other than the port(s) in the fluid management chamber at the microporated site. The side layer or wall of the fluid management chamber opposite these ports could be constructed with one or more very small opening(s) to create a vent allowing the biological fluid to fully fill the fluid management chamber, yet minimize the exposed surface of the biological fluid when the assay area is full, thereby reducing evaporation. The reduction of evaporative losses is more significant when using a vacuum-induced harvesting process because the rarefied atmosphere will accelerate any evaporation process. Experiments have shown that simply keeping the volume of the chamber small, and providing a capillary type channel (comprised of the sensor on one side and a layer on the other with or without the optional wicking mesh therebetween) for the biological fluid to enter upon exiting the body, evaporative losses can consistently be kept under 5% over a 45 second harvesting cycle. A large chamber and an exposed bead of biological fluid on the surface of the skin can allow up to 30% of the biological fluid to evaporate during this same 45 second interval, under the same temperature conditions.

An additional feature of pneumatically sealing the integrated device is that by virtue of its contact with the tissue, these portions of the integrated assay system maintain the mechanical alignment of the micropore(s) in the tissue with the inlet ports of the integrated device.

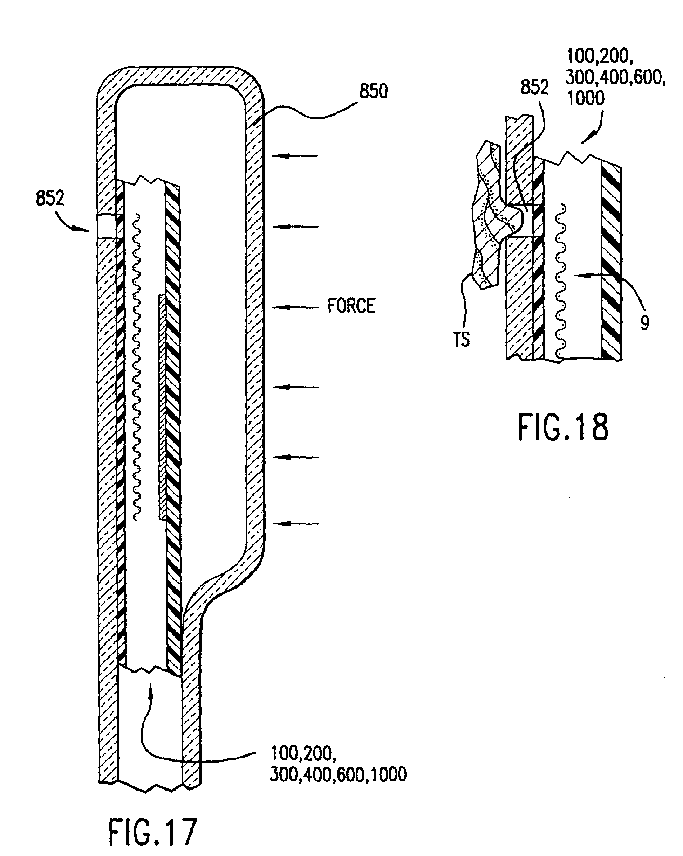

FIGs. 17 and 18 illustrate the use of a mechanical system to apply positive pressure to the integrated device. A mechanical element 850 is provided, having a small opening 852, 2 mm to 4 mm in diameter. The mechanical element 850 permits the integrated device to slide between two opposing surfaces and contains the integrated device. Applying force to the mechanical element 850 presses the integrated device onto the skin at the poration site and thus creates a positive pressure gradient in the biological fluid harvested from the tissue TS, i.e., the skin, forcing it towards the micropores where it can exit the tissue and enter the inlet port(s) of the fluid management chamber of the integrated device (100, 200, 300, 400, 600, 1000). The tissue bulges into the opening 852 as shown in FIG. 18. A close registration is maintained between the inlet ports to the integrated device and the micropores, which have been, or simultaneously will be, formed in the tissue directly beneath these ports. The mechanical device 850 may be optically clear on its top portion to allow for optical thermal ablation and optical reading of the photometric sensor in that form of the integrated device.

The application of mechanically induced pressure may be continuous, modulated as in a sine or triangle wave, or pulsed. The rate and modulation pattern may be optimized to take advantage of the fluidic properties of the skin tissues such as the local permeabilities, and the refill or recovery rates of the tissue once some portion of the biological fluid has been pressed out of it. Clinical experiments have demonstrated that applying a few pounds per square inch of pressure to the skin with a flat plate having a 2 mm to 4 mm diameter hole in it surrounding the micropore(s) rapidly forces biological fluid to exit the pores and pool on the surface of the skin. In addition, the use of the mechanical device may be combined with vacuum to provide an additional biological fluid forcing function, and to possibly assist in the fluid management of the biological fluid as it exits the body. A further benefit of applying firm pressure to the system during the thermal poration process is that this pressure helps ensure a good thermal connection between the heat probe created by the optically heated absorber targets and the skin to be porated.

One important requirement of any integrated microporation, harvesting, assay system is that the input ports or channels to the assay system be in physical registration or alignment with the micropores on the skin to ensure an efficient transfer of fluid from the micropores to the assay strip. Registration and alignment can be achieved by employing an adhesive or tacky silicone product to temporarily attach the integrated device. Alternatively, registration and alignment can be accomplished by installing the assay strip component within a translation system which, when activated, brings the input ports or channels of the assay strip into close enough proximity to the biological fluid exiting the micropores to cause the directed flow of this biological fluid into the assay strip. This sort of translation can be achieved in a number of ways such as, but not limited to, a small servo motor activated by a controller to move the assay strip into position at the appropriate time; a pneumatically positioned system driven by the same vacuum source described in conjunction with FIGs. 15 and 16; or a system design wherein the flexure of the skin itself under either the vacuum or pressure as described above brings the biological fluid on the surface of the skin into contact with the assay strip. An additional advantage of the translation system in the fluid management portion of the integrated microporation, harvesting, assay system is that it can be designed to supply the entire required fluid sample in a bolus delivery to the assay system, rather than trickling it over some longer period of time. In many cases a bolus delivery of sample fluid enables a more accurate assay to be conducted using standard disposable assay strip design concepts.

Furthermore, by designing the integrated microporation, harvesting, assay system in such a manner that the biological fluid management is handled with minimal dead space outside of the active region of the biosensor, a system can be built which uses very small samples of biological fluid to obtain an accurate assay of a selected analyte. Tests have been conducted on commercially available systems using glucose sensing amperometric biosensors that incorporated all of these features and it was found that the glucose concentration in a sample of biological fluid smaller than 1/3 of a microliter could be quantified, by modifying commercially available glucose test strips. One of the additional advantages gained by using interstitial fluid as the fluid sample for the assay system is the almost total lack of red blood cells in the sample. Most commercial strip based assay systems utilize some means of separating the corpuscular component from a whole blood sample prior to the application of the fluid sample to the assay element. In many cases, this process is performed by the use of some sort of wicking mesh designed to trap the blood cells and let only the serum move through to the assay area. These filtering approaches can use up as much as 4/5 of the original sample volume in the process. By using interstitial fluid, this step is no longer needed. In other words, a typical sample size of 3 to 10 microliters is normally required for a blood based glucose monitoring disposable assay strip design whereas by utilizing the ability to place an unfiltered interstitial fluid sample directly on the active reagent treated portion of an assay system, it has been demonstrated that quantitative readings of a selected analyte can be obtained with fluid samples as small as 1/3 µL of interstitial fluid using modified conventional disposable assay strip technologies.

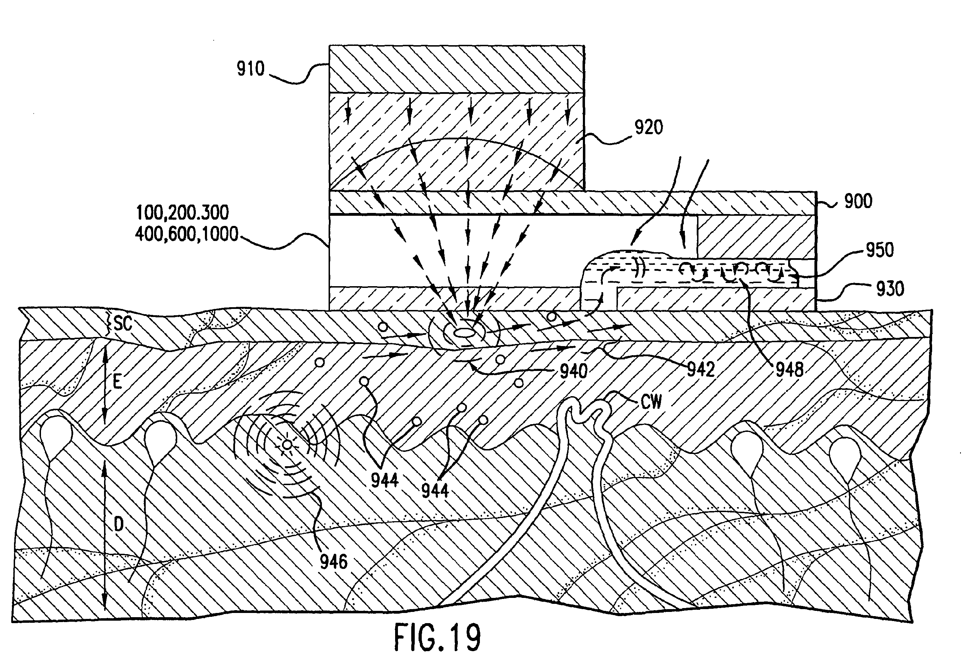

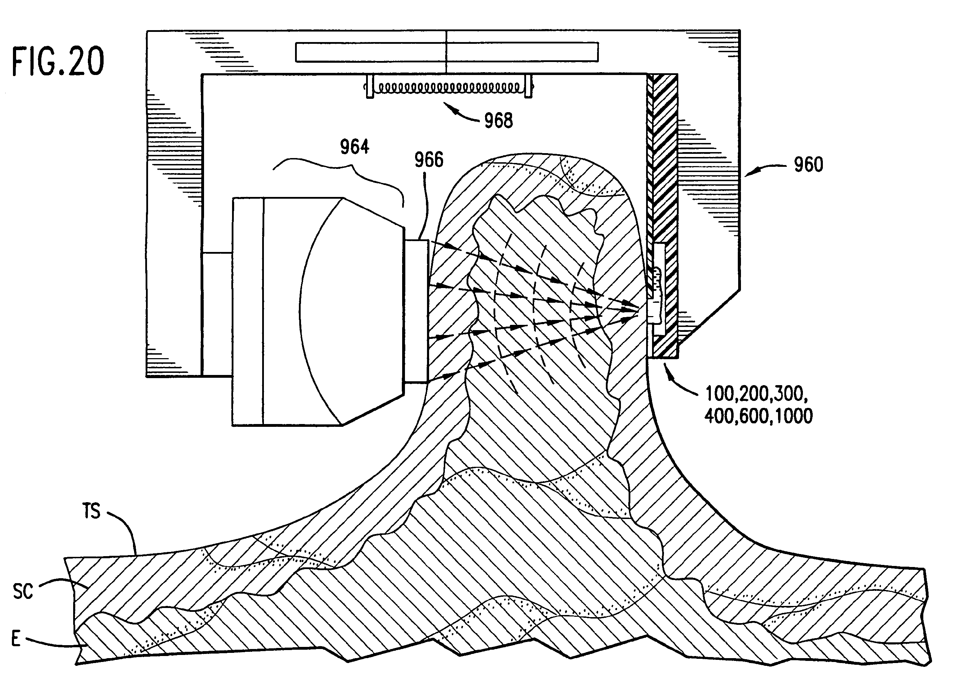

Turning to FIGs. 19 and 20, the use of sonic energy in conjunction with the integrated device will be described. The integrated device can be used in conjunction with a means for coupling sonic energy from a transducer into the system and optionally into the tissues upon which the integrated system is disposed. In particular, experiments have shown that sonic energy in the range of 5 kHz to 30 MHz can be useful to enhance the outflux of biological fluid from a microporated area of skin. Furthermore, the literature on the use of sonic energy supports the extension of the useable frequencies as high as 500 MHz.

The permeation enhancing effect of sonic energy is due to several different mechanisms in the tissue, including but not limited to, the acoustic streaming induced in the fluids within the skin tissues, the directable effects of the sonic radiation pressure which can act directly to push the fluid is a desired direction, the reduction in the viscosity of the fluid itself, the modification of the surface tension effects both within the tissues and at the surface of the micropore, the local heating possible from the absorption of the sonic energy and the body's natural edemic response to this, the opening of microscopic temporary channels in the various membranes and layers within the tissue such as the capillary and vessel walls, the effect on these tissue structures due to cavitation achieved with selected frequencies and intensities of sonic energy, and the simple physical shaking of the system possible with various pulsed and modulated patterns of sonic energy, and the like.

When incorporating a sonic energy source into a system such as this, it is important to consider the acoustic impedance of the various layers through which the sound waves travel, and the matching of the acoustic impedance at the interfaces of the various layers. For diagnostic ultrasound, a gel is frequently used to facilitate the coupling of the sonic energy into the tissue and this approach could be used to mate the bottom surface of the integrated device element to the surface of the tissue, such as skin. An alternative solution to the coupling issue that eliminates the need for a coupling gel, is to use an appropriately designed gasket type of material, such as a silicone or hydrogel to form the sonic connection. In addition, tacky or adhesive elements are useful to both seal a fluid management chamber and maintain registration between the micropores and the inlet port of the assay system. These elements are also useful as efficient acoustic coupling agents.

In the case where a focused acoustic field is desired, multiple selectively phased sources, sonic lenses or reflectors could all be employed to generate the desired energy distribution within the target zone. A purposefully created impedance mismatch within the media through which the sound waves propagate can be used as a means of forming a reflective boundary. Basically, all traditional wave propagation equations hold true for sonic energy, just as they do for electromagnetic energy, and as such the same type of wave guide or energy directing methods can be employed to focus the sonic energy where desired.

The schematic representation in FIG. 19 shows an integrated device (100, 200, 300. 400, 600. 1000) having a compliant layer 900 placed on the top to form an efficient coupling for sonic energy. The sonic energy is generated by sonic energy generation means, such as a piezo-electric transducer 910. A sonic lens element 920 is placed between the piezo-electric transducer 910 and the compliant layer 900. A coupling gasket 930 may also be provided to pneumatically seal the integrated device to the surface of the tissue (with optional application of suction) and to assist in the acoustic coupling of the sonic energy.

The acoustic waves can be optimized to have any of several recognized actions and effects on the performance of the harvesting and analysis of biological fluid, or delivery of bio-active agents. The sonic energy can be propagated through the integrated device, through the coupling gasket 930, to the tissue (such as skin), wherein SC denotes the stratum corneum, E denotes the epidermis and D denotes the dermis.

Within the tissue, the direct effects of the sonic energy include local warming of the tissue through the direct absorption of the sonic energy. This is shown at reference numeral 940. Depending on the frequency selection and possible modulations of the frequency and amplitude of the sonic energy, an acoustic streaming effect can be achieved within the tissue, accelerating the fluidic movement between cells and within cells and vessels. This is shown at reference numeral 942. The amount of increase in the local velocity of the fluid has been shown to be more than one order of magnitude using visible tracers in *in vivo* real-time video microscopy experiments.

Similarly, when the frequency and intensity and possible modulation thereof are selected appropriately, a cavitation effect shown by cavitation bubbles at reference numeral 944, is achieved which can have substantial secondary effects on the tissue properties due to possible microscopic shearing of some tissue structures, the transitory opening up of micro-porous sites in various membranes such as the capillary walls CW within the tissue, and other effects due to the shock waves, shown at reference numeral 946, created upon the collapse of the cavitation bubble.

The presence of the acoustic vibrations within the fluid management chamber of the integrated device itself can also be used to enhance the motion of the fluid. These effects can be due to a directed radiation pressure gradient shown at reference numeral 948 which can be created by proper alignment and focusing of the sonic energy, the enhancement of capillary transport action shown at reference numeral 950 by the acoustic energy, the active out-gassing of dissolved gas in the fluid which can help to eliminate error causing bubbles in the active assay area of the system, and the localized and chaotic micro-fluidic vortices shown at reference numeral 950 created within the fluid management chamber which can be used to reduce the required assay reaction time by eliminating the dependency on passive diffusion effects and thereby evenly distribute the reactive process within the sample.

The activation of the sonic energy source can be selectively controlled to work in a coordinated fashion with the other components of the system, even to the point of operating with significantly different parameters during different portions of the poration, harvesting, assay process. For example, a sequence of sonic energy use is:
1. Start with a controlled burst of higher energy ultrasound designed to temporarily permeabilize the capillary walls and the intervening bulk tissue structures during the poration cycle. The presence of this type of short pulse of high intensity sonic energy has also been shown to reduce the perceived sensation associated with the thermal poration process by most subjects.
2. During the fluid collection phase, a lower power, swept frequency modulation setting of the sonic energy could be used to induce the acoustic streaming effect within the tissue designed to bring more biological fluid to the surface.
3. As the biological fluid exits the body and enters the inlet port of the assay system (the integrated device), the sonic energy could be re-tuned to more optimally enhance the surface tension driven transport of the biological fluid towards the active reagent area. Biological fluid transport could be used both within a capillary channel, a mesh or a porous media transport layer system.
4. Once on the active reagent layer, the operating parameters of the sonic energy could once again be adjusted to create the active "stirring" of the fluid within the fluid management chamber to facilitate a more rapid and/or accurate quantification of the selected analyte.

Essentially all of the same functional modalities described in conjunction with FIG. 19 can also be realized with an alternative configuration wherein a remotely placed sonic source is used to direct the acoustic energy towards the desired portion of the assay element of the integrated device by beaming it through a fold of intervening flesh.

With reference to FIG. 20, a clamp assembly 960 is provided to pinch a fold of tissue, such as skin between a transducer assembly. The transducer assembly comprises an acoustic transducer 962, a focusing element 964, and a coupling layer 966. The integrated device (100, 200, 300, 400 and 600) is at an opposite side of the pinch of skin. The dimensions of the clamp assembly 960 are such that when the tensioning device 968 pulls the two clamp halves together, they hit a hard stop and the spacing from the face of the transducer assembly and the inlet port of the fluid management chamber of the integrated device is positioned at an optimal position in {x, y, and z}coordinates to coincide with the sonic energy fields as desired. For example, FIG. 20 shows the focal point of the sonic field is roughly coincident with the inlet port of the assay chamber, which may be one selected mode of operation. However, by shifting the frequency of the sound waves, this focal point can be moved in and out from the face of the transducer.

Experiments have shown that it can be advantageous to modulate the frequency, thereby shifting the sonic energy field position and local intensities. This sort of control of sonic energy fields has been shown to induce an active pumping action at the modulation rate of the system which can similarly be used to exploit certain fluid and mechanical properties of the tissues.

By employing a clamping mechanism, which forces the sonic transducer against the skin surface, the coupling losses at this interface can be reduced and/or controlled within a design specification.

The initial deflection into the inter-clamp space can be accomplished by placing the entire assembly within a suction system, such as that shown in FIGs. 15 and 16, which pulls the flesh into the space, and as the vacuum increases, provides the clamping force to pull the two halves of the clamp assembly together to the stops. Similarly this could be accomplished via mechanically feeding a pinch of skin into the space and then letting the clamp grab the tissue.

An additional function of sonic energy applicable to all of the previously discussed sonic enhancement concepts is the demonstrated beneficial effects it can have on the wound healing process. Clinical results have consistently shown positive effects when sonic energy is applied to various types of wounds including bums and other superficial skin traumas. In the case of microporation created in the outer layers of the skin, this acceleration of the healing process can be exploited to improve the overall acceptance of the system by the end user and health care practitioners.

In summary, the present invention is directed to an integrated poration harvesting and analysis device, comprising a first layer having a porating element disposed thereon, the porating element forming at least one opening in the tissue; a sensor positioned in fluid communication with the at least one opening in the tissue, the sensor being responsive to a biological fluid collected from the tissue to provide an indication of a characteristic of the biological fluid. Similarly, the present invention is directed to an integrated fluid harvesting and analyis device comprising a first layer for positioning in contact with tissue and through which poration of tissue is achieved such that at least one opening is formed in the first layer and at least one opening is formed in the tissue; a sensor positioned in fluid communication with the at least one opening of the first layer, the sensor being responsive to a biological fluid collected from the tissue to provide an indication of a characteristic of the biological fluid. A quantity of photothermal material is disposed on a portion of the first layer, which is heated by optical energy to form the micropore (opening) in the tissue, and thereby create the opening in the first layer.

A counterpart method for harvesting biological fluid from tissue and analyzing the biological fluid is provided comprising steps of placing a layer in contact with a surface of tissue; forming at least one hole in the tissue; collecting biological fluid from the tissue through at least one opening in the layer; and wetting a sensor that is positioned in fluid communication with the at least one opening in the layer with biological fluid to measure a characteristic of the biological fluid. The means to form the opening may be a mechanical element (lancet, needle, etc.), an electrically heated poration element, an optically heated poration element, etc. In addition, the opening in the tissue may be formed adjacent to an edge of the layer, whereby biological fluid enters the layer through a capillary feed channel formed between the top and bottom layers of the device, as described above.

Various modifications and alterations of this invention will become apparent to those skilled in the art without departing from the scope of this invention as defined by the appended claims, and it should be understood that this invention is not to be unduly limited to the illustrative embodiments set forth herein.

## Claims

1. An integrated fluid harvesting and analysis device (100, 200, 300, 400, 600, 1000) for obtaining a biological fluid sample from a tissue and analyzing a characteristic thereof, said device comprising:
a first layer (110, 210, 310) having a porating element (130, 240, 324, 450, 640, 1030) disposed thereon, the porating element being adapted to form at least one opening in the tissue;
a sensor (120, 230, 340, 420, 630, 1020) adapted to be positioned in fluid communication with the at least one opening in the tissue, said sensor being responsive to the biological fluid collected via the at least one opening in the tissue to provide an indication of a characteristic of the biological fluid;
a fluid management chamber which facilitates the movement of the biological fluid from the at least one opening to the sensor; and
**characterised in that** a chemical substance in the fluid management chamber actively promotes the movement of the biological fluid towards the sensor by manipulating the surface tension of the biological fluid.

2. The device of claim 1, wherein the fluid management chamber contains a wicking mesh (140, 250, 440, 620) to enhance the conductivity of the biological fluid towards the sensor.

3. The device of claim 2, wherein the wicking mesh comprises a surfactant compound.

4. The device of claim 1, wherein the fluid management chamber is in the form of a capillary channel.

5. The device of any of claims 1 to 4, wherein the fluid management chamber further comprises a mechanical drive means (280) that acts upon the fluid management chamber to move the biological fluid towards the sensor.

6. The device of claim 5, wherein the mechanical drive means comprises a cam or roller mechanism (280).

7. The device of any of claims 1 to 6, wherein the interior of the fluid management chamber is covered with a surfactant compound.

8. The device of any of the preceding claims, further comprising a second layer overlying the first layer, the sensor being positioned between the first layer and the second layer.

9. The device of claim 8, wherein said second layer comprises a portion which is transparent to optical energy,

10. The device of any of the preceding claims, wherein the porating element is a heat conducting element that is heatable such that the temperature of tissue-bound water and other vaporizable substances in a selected area of the surface of the tissue proximate the heat conducting element is elevated above the vaporization point of water and other vaporizable substances thereby removing the surface of the tissue in said selected area.

11. The device of claim 10, wherein the porating element comprises a photothermal material (240, 450) that is responsive to application of optical energy to heat up and conduct heat to the surface of the tissue for forming at least one opening therein.

12. The device according to any of claims 1 to 9, wherein the porating element comprises at least one mechanical puncturing member (1030) protruding from a bottom surface of the first layer.

13. The device of any of the preceding claims, wherein the sensor comprises an electrochemical biosensor which is responsive to a level of glucose in interstitial fluid.

14. The device according to any of claims 1 to 12, wherein the sensor comprises a colorimetric sensor that provides an indication of glucose level in interstitial fluid.

15. The device of any of the preceding claims, further comprising a sonic transducer formed of a compliant sonic transmissive material positioned above the layer to deliver sonic energy to the tissue surrounding a location of the tissue where the at least one opening is to be formed.

16. The device of claim 15, wherein the sonic transducer is formed of a compliant silicone material.

17. The device of claim 15 or 16, comprising at least two sonic transducers positioned on opposite sides of the sensor.

18. The device of any claims 1 to 9, wherein the porating element comprises at least one electrically energized heat conducting element.

19. The device of claim 18, further comprising at least two conductors embedded in the tissue-contacting layer and the at least one electrically energized heat conducting element being connected to the conductors for supplying electric current to the at least one electrically heatable element.

20. The device of claim 1, further comprising a mechanical element having a small opening therein and capable of receiving the integrated device such that the porating element is aligned with the small opening, the mechanical element responsive to downward force thereon to cause the surface of the tissue to bulge into the small opening.

21. The device of claim 1, further comprising sealing means for pneumatically sealing the integrated device to the surface of the tissue and forming a sealed chamber, and means coupled to the sealing means for supplying negative pressure to the sealed chamber.

22. The device of claim 21, and further comprising a sealed electrical connection to the sensor and/or probe via the sealing means.

23. The device of claim 21, wherein surface portions of the first layer are coated with hydrophobic substances.

24. The device of any of the preceding claims, further comprising a sense electrode coupled to the sensor to facilitate determination that the sensor is sufficiently wetted with biological fluid.

25. The device of claim 1, further comprising means for coupling sonic energy through the device to the tissue.

26. The device of claim 25, and further comprising control means for controlling parameters of the sonic energy so that the sonic energy is adjusted to optimize each stage of a harvesting and analysis process.

## Patentansprüche

1. Integrierte Flüssigkeitssammel- und Analysevorrichtung (100, 200, 300, 400, 600, 1000) zum Gewinnen einer biologischen Flüssigkeitsprobe aus einem Gewebe und zum Analysieren einer Eigenschaft hiervon, wobei die Vorrichtung umfasst:
- eine erste Schicht (110, 210, 310) mit einem darauf angeordneten Porationselement (130, 240, 324, 450, 640, 1030), wobei das Porationselement dazu geeignet ist, wenigstens eine Öffnung in dem Gewebe zu bilden,
- einen Sensor (120, 230, 340, 420, 630, 1020), der dazu geeignet ist, in Flüssigkeitsverbindung mit der wenigstens einen Öffnung im Gewebe angeordnet zu werden, wobei der Sensor auf die über die wenigstens eine Öffnung in dem Gewebe gesammelte biologische Flüssigkeit anspricht, um einen Hinweis auf eine Eigenschaft der biologischen Flüssigkeit zu liefern,
- eine Flüssigkeitsmanagementkammer, die die Bewegung der biologischen Flüssigkeit von der wenigstens einen Öffnung zum Sensor erleichtert, und
- **dadurch gekennzeichnet ist, dass** eine chemische Substanz in der Flüssigkeitsmanagementkammer die Bewegung der biologischen Flüssigkeit in Richtung auf den Sensor durch Beeinflussen der Oberflächenspannung der biologischen Flüssigkeit aktiv fördert.

2. Vorrichtung nach Anspruch 1, wobei die Flüssigkeitsmanagementkammer ein Netzgewebe (140, 250, 440, 620) enthält, um die Leitfähigkeit der biologischen Flüssigkeit in Richtung Sensor zu steigern.

3. Vorrichtung nach Anspruch 2, wobei das Netzgewebe eine oberflächenaktive Verbindung umfasst.

4. Vorrichtung nach Anspruch 1, wobei die Flüssigkeitsmanagementkammer in Form eines Kapillarkanals vorliegt.

5. Vorrichtung nach einem der Ansprüche 1 bis 4, wobei die Flüssigkeitsmanagementkammer ferner mechanische Antriebsmittel (280) umfasst, die auf die Flüssigkeitsmanagementkammer einwirken, um die biologische Flüssigkeit in Richtung Sensor zu bewegen.

6. Vorrichtung nach Anspruch 5, wobei die mechanischen Antriebsmittel einen Nocken- oder Walzenmechanismus (280) umfassen.

7. Vorrichtung nach einem der Ansprüche 1 bis 6, wobei das Innere der Flüssigkeitsmanagementkammer mit einer oberflächenaktiven Verbindung bedeckt ist.

8. Vorrichtung nach einem der vorhergehenden Ansprüche, ferner umfassend eine zweite Schicht, die die erste Schicht überlagert, wobei der Sensor zwischen der ersten und der zweiten Schicht angeordnet ist.

9. Vorrichtung nach Anspruch 8, wobei die zweite Schicht einen Abschnitt umfasst, der gegenüber optischer Energie transparent ist.

10. Vorrichtung nach einem der vorhergehenden Ansprüche, wobei das Porationselement ein wärmeleitendes Element ist, das erwärmt werden kann, so dass die Temperatur von gewebegebundenem Wasser und anderen verdampfbaren Substanzen in einem ausgewählten Bereich der Gewebeoberfläche nahe dem wärmeleitenden Element über den Verdampfungspunkt von Wasser und anderen verdampfbaren Substanzen angehoben wird, wodurch die Oberfläche des Gewebes in dem ausgewählten Bereich entfernt wird.

11. Vorrichtung nach Anspruch 10, wobei das Porationselement ein photothermales Material (240, 450) umfasst, das auf die Anwendung von optischer Energie zum Aufwärmen anspricht und Wärme an die Oberfläche des Gewebes leitet, um wenigstens eine Öffnung darin zu bilden.

12. Vorrichtung nach einem der Ansprüche 1 bis 9, wobei das Porationselement wenigstens ein mechanisches Durchstoßteil (1030) umfasst, das aus einer Bodenfläche der ersten Schicht vorsteht.

13. Vorrichtung nach einem der vorhergehenden Ansprüche, wobei der Sensor einen elektrochemischen Biosensor umfasst, der auf einen Glukosespiegel in der Zwischenzell-Flüssigkeit anspricht.

14. Vorrichtung nach einem der Ansprüche 1 bis 12, wobei der Sensor einen kolorimetrischen Sensor umfasst, der einen Hinweis auf den Glukosespiegel in der Zwischenzell-Flüssigkeit liefert.

15. Vorrichtung nach einem der vorhergehenden Ansprüche, ferner umfassend einen Schallwandler, gebildet aus einem über der Schicht angeordneten nachgiebigen schalldurchlässigen Material, um Schallenergie an das Gewebe zu leiten, das eine Stelle des Gewebes umgibt, wo die wenigstens eine Öffnung gebildet werden soll.

16. Vorrichtung nach Anspruch 15, wobei der Schallwandler aus einem nachgiebigen Silikonmaterial gebildet ist.

17. Vorrichtung nach Anspruch 15 oder 16, umfassend wenigstens zwei Schallwandler, die auf gegenüberliegenden Seiten des Sensors angeordnet sind.

18. Vorrichtung nach einem der Ansprüche 1 bis 9, wobei das Porationselement wenigstens ein elektrisch erregtes wärmeleitendes Element umfasst.

19. Vorrichtung nach Anspruch 18, ferner umfassend wenigstens zwei in die gewebeberührende Schicht eingebettete Leiter und das wenigstens eine elektrisch erregte wärmeleitende Element, das mit den Leitern verbunden ist, um dem wenigstens einen elektrisch erwärmbaren Element elektrischen Strom zuzuführen.

20. Vorrichtung nach Anspruch 1, ferner umfassend ein mechanisches Element mit einer kleinen Öffnung und geeignet zur Aufnahme der integrierten Vorrichtung, so dass das Porationselement mit der kleinen Öffnung fluchtet, wobei das mechanische Element auf eine hierauf nach unten wirkende Kraft anspricht, um die Oberfläche des Gewebes in die kleine Öffnung aufzuwölben.

21. Vorrichtung nach Anspruch 1, ferner umfassend Abdichtungsmittel, um die integrierte Vorrichtung mit Druckluft gegenüber der Oberfläche des Gewebes abzudichten und eine abgedichtete Kammer zu bilden, sowie mit den Abdichtungsmitteln gekoppelte Mittel, um der abgedichteten Kammer Unterdruck zuzuführen.

22. Vorrichtung nach Anspruch 21, und ferner umfassend eine abgedichtete elektrische Verbindung zu Sensor und/oder Sonde über die Abdichtungsmittel.

23. Vorrichtung nach Anspruch 21, wobei Oberflächenabschnitte der ersten Schicht mit hydrophoben Substanzen beschichtet sind.

24. Vorrichtung nach einem der vorhergehenden Ansprüche, ferner umfassend eine mit dem Sensor verbundene Abfrageelektrode, um leichter bestimmen zu können, dass der Sensor ausreichend mit biologischer Flüssigkeit benetzt ist.

25. Vorrichtung nach Anspruch 1, ferner umfassend Mittel, um Schallenergie durch die Vorrichtung mit dem Gewebe zu koppeln.

26. Vorrichtung nach Anspruch 25, und ferner umfassend Steuerungsmittel, um Parameter der Schallenergie zu steuern, so dass die Schallenergie angepasst wird, um jede Stufe eines Sammel- und Analyseverfahrens zu optimieren.

## Revendications

1. Dispositif intégré pour le prélèvement d'un fluide et son analyse (100, 200, 300, 400, 600, 1000) destiné à obtenir un échantillon de fluide biologique à partir d'un tissu et analyser une caractéristique de celui-ci, ledit dispositif comprenant :
- une première couche (110, 210, 310) ayant un élément de formation de pores (130, 240, 324, 450, 640, 1030) disposé sur celle-ci, l'élément de formation de pores étant adapté pour former au moins une ouverture dans le tissu;
- un capteur (120, 230, 340, 420, 630, 1020) adapté pour être positionné en communication fluidique avec la au moins une ouverture dans le tissu, ledit capteur étant sensible au fluide biologique recueilli par la au moins une ouverture dans le tissu pour fournir une indication d'une caractéristique du fluide biologique ;
- une chambre de gestion de fluide qui facilite le mouvement du fluide biologique depuis la au moins une ouverture vers le capteur ; et
**caractérisé en ce qu'**une substance chimique dans la chambre de gestion de fluide favorise activement le mouvement du fluide biologique en direction du capteur en manipulant la tension superficielle du fluide biologique.

2. Dispositif selon la revendication 1, dans lequel la chambre de gestion de fluide contient une mèche conductrice (140, 250, 440, 620) pour améliorer la conductivité du fluide biologique en direction du capteur.

3. Dispositif selon la revendication 2, dans lequel la mèche conductrice comprend un composé tensioactif.

4. Dispositif selon la revendication 1, dans lequel la chambre de gestion de fluide a la forme d'un canal capillaire.

5. Dispositif selon l'une quelconque des revendications 1 à 4, dans lequel la chambre de gestion de fluide comprend en outre un moyen d'entraînement mécanique (280) qui agit sur la chambre de gestion de fluide pour déplacer le fluide biologique en direction du capteur.

6. Dispositif selon la revendication 5, dans lequel le moyen d'entraînement mécanique comprend un mécanisme à cames ou à rouleaux (280).

7. Dispositif selon l'une quelconque des revendications 1 à 6, dans lequel l'intérieur de la chambre de gestion de fluide est recouvert d'un composé tensioactif.

8. Dispositif selon l'une quelconque des revendications précédentes, comprenant en outre une deuxième couche recouvrant la première couche, le capteur étant positionné entre la première couche et la deuxième couche.

9. Dispositif selon la revendication 8, dans lequel ladite deuxième couche comprend une partie qui est transparente à l'énergie optique.

10. Dispositif selon l'une quelconque des revendications précédentes, dans lequel l'élément de formation de pores est un élément conduisant la chaleur qui est apte à être chauffé de manière à ce que la température de l'eau liée aux tissus et d'autres substances vaporisables dans une zone choisie de la surface du tissu à proximité de l'élément conduisant la chaleur soit élevée au-dessus du point de vaporisation de l'eau et d'autres substances vaporisables, retirant de cette manière la surface du tissu dans ladite zone choisie.

11. Dispositif selon la revendication 10, dans lequel l'élément de formation de pores comprend une matière photothermique (240, 450) qui est sensible à l'application d'une énergie optique pour se réchauffer et conduire la chaleur vers la surface du tissu pour former au moins une ouverture dans celui-ci.

12. Dispositif selon l'une quelconque des revendications 1 à 9, dans lequel l'élément de formation de pores comprend au moins un élément de ponction mécanique (1030) faisant saillie depuis une surface inférieure de la première couche.

13. Dispositif selon l'une quelconque des revendications précédentes, dans lequel le capteur comprend un biocapteur électrochimique qui est sensible à un niveau de glucose dans un liquide interstitiel.

14. Dispositif selon l'une quelconque des revendications 1 à 12, dans lequel le capteur comprend un capteur colorimétrique qui fournit une indication du niveau de glucose dans un fluide interstitiel.

15. Dispositif selon l'une quelconque des revendications précédentes, comprenant en outre un transducteur acoustique formé d'un matériau souple transmettant les sons positionné au-dessus de la couche pour délivrer une énergie acoustique au tissu entourant un endroit du tissu où la au moins une ouverture doit être formée.

16. Dispositif selon la revendication 15, dans lequel le transducteur acoustique est formé d'un matériau souple de silicone.

17. Dispositif selon la revendication 15 ou 16, comprenant au moins deux transducteurs acoustiques positionnés sur des côtés opposés du capteur.

18. Dispositif selon l'une quelconque des revendications 1 à 9, dans lequel l'élément de formation de pores comprend au moins un élément conduisant la chaleur alimenté électriquement.

19. Dispositif selon la revendication 18, comprenant en outre deux conducteurs incrustés dans la couche en contact avec le tissu et le au moins un élément conduisant la chaleur alimenté électriquement qui est connecté aux conducteurs pour alimenter en courant électrique le au moins un élément apte à être chauffé électriquement.

20. Dispositif selon la revendication 1, comprenant en outre un élément mécanique ayant une petite ouverture dans celui-ci et étant apte à recevoir le dispositif intégré de sorte que l'élément de formation de portes est aligné avec la petite ouverture, l'élément mécanique étant sensible à une force descendante sur celui-ci pour faire que la surface du tissu forme une saillie dans la petite ouverture.

21. Dispositif selon la revendication 1, comprenant en outre un moyen d'étanchéité pour réaliser l'étanchéité pneumatique du dispositif intégré sur la surface du tissu et former une chambre hermétique, et un moyen couplé au moyen d'étanchéité pour fournir une pression négative à la chambre hermétique.

22. Dispositif selon la revendication 21, et comprenant en outre une connexion électrique hermétique avec le capteur et/ou la sonde par le moyen d'étanchéité.

23. Dispositif selon la revendication 21, dans lequel des parties superficielles de la première couche sont revêtues de substances hydrophobes.

24. Dispositif selon l'une quelconque des revendications précédentes, comprenant en outre une électrode d'excitation couplée au capteur pour aider à déterminer su le capteur est suffisamment mouillé par le fluide biologique.

25. Dispositif selon la revendication 1, comprenant en outre un moyen pour coupler l'énergie acoustique parle dispositif au tissu.

26. Dispositif selon la revendication 25, et comprenant en outre un moyen de commande pour commander des paramètres de l'énergie acoustique de sorte que l'énergie acoustique soit ajustée pour optimiser chaque étape d'un processus de prélèvement et d'analyse.
